# EUROPEAN PATENT APPLICATION

(11) **EP 2 338 500 A1**
(43) Date of publication of application: **29.06.2011**
(21) Application number: 09425529.6
(22) Date of filing: 23.12.2009
(51) Int. Cl.: A61K 36/63

(54) **Process for producing concentrated and refined actives from tissues and byproducts of Olea europaea with membrane technologies**

(71) Applicant: Phenofarm S.r.l., 00195 Roma (IT); Romani, Annalisa, 51031 S.Michele Agliana (IT); Pizzichini, Massimo, 00123 Roma (IT)
(72) Inventor: Pizzichini, Daniele, 00123 Roma (IT); Russo, Claudio, 00186 Roma (IT); Vitagliano, Massimo, 80022 Arzano (NA) (IT); Pizzichini, Massimo, 00123 Roma (IT); Romani, Annalisa, 51031 S. Michele Agliana (PT) (IT); Ieri, Francesca, 50131 Firenze (IT); Pinelli, Patrizia, 50127 Firenze (IT); Vignolini, Pamela, 51100 Pistoia (IT)
(74) Representative: Banchetti, Marina

(57) **Abstract**

The invention concerns an integrated process affording the production of purified fractions, as powder or concentrated solution, consisting of different biologically active compounds and families of compounds, starting from olive tree tissues and wastes of the olive oil industry and olive tree cultures, in particular olive tree leaves and twigs (pruning wastes), residues from the oil production (pomace or husks) and pigmented olive pulp. The refined products obtainable comprise not only oleuropein, hydroxytyrosol and related compounds, but also verbascoside and red anthocyanoside pigments.

The proposed process is based on the integration of hot or cold extraction from the various vegetal starting materials with separation techniques by membrane tangential filtration, specifically microfiltration (MF) or ultrafiltration (UF), optionally followed by nanofiltration (NF) and completed by reverse osmosis (Ol). These operations are completed, where necessary, by refining treatments of the thus obtained fractions of UF retentate, NF retentate and Ol retentate on chromatographic resins.

## Description

The present invention relates to a process for producing concentrated and refined actives from tissues and by-products of *Olea europaea L.* with membrane technologies and adsorption on chromatographic resins. More specifically, the invention concerns an integrated process affording purified fractions as powders or concentrated solutions, consisting of different biologically active compounds and families of compounds, starting from olive tree tissues yielded by olive cultivation and from olive oil industry residues.

As it is known, olive growing represents for the whole Mediterranean area, and in particular for Italy, a productive sector of a primary importance. In Italy, olive is spread over a mostly hilly area which represents about one fifth of the world surface reserved to olive tree cultivation. However, this sector is hindered by a heavy generation of by-products, substantially falling into one of the following groups: 1) vegetal tissues (leaves and pruning wastes); 2) true oil production residues (olive mill wastes: vegetation waters, wet and exhaustted olive husks or pomace).

The latter class of products is characterized by a strong pollution load, and therefore requires appropriate procedures for its disposal in accordance with the current environmental laws (i.e., for Italy, Decree-law No. of 11/11/96)

Also the vegetal tissues of the olive tree (i.e. leaves and twigs resulting from pruning) bring about a disposal problem, although less serious than the problem represented by the disposal of vegetation waters: Also in that case appropriate measures or treatments are required in order to be allowed to release the waste material in the environment.

The use of wastes and residues from the oil production chain may allow to generate economical resources starting from non-conventional starting materials (e.g., wet pomace, vegetation waters, destoned exhausted pomace, vegetal tissues), by exploiting the commercialization of the active ingredients in sectors different form the oil industry, such as those of cosmetic and phytotherapeutic products, in both cases with a high added value.

It is reasonable to hypothesize that the earnings deriving from the exploitation of olive industry by-products may provide the resources needed to re-launch the whole sector of the extra-virgin oil with respect to productions of a lower quality. Actually, in the latest years the scientific world has shown a renewed interest towards the possible alternative or additional uses of the tissues of the *Olea europaea* plant, in particular as concerns the cosmetic and therapeutic applications of the herbal preparations obtained from olive leaves.

The olive leaves from pruning represent a huge waste of the olive growing/oil production sector, whose impact is evident if it is considered that each olive tree produces from 30 to 60 kg per year of pruning waste, of which, on average, 10-15% are leaves and 85-90% are branches. As concerns the olive mill wastes, on the other hand, the amounts of leaves and twigs accompanying the olives supplied to the mill, which are to be separated therefrom before processing the olives, are about 3% - 4% by weight of the olives treated.

The present technology is mainly oriented towards converting the waste of the olive growing/oil production sector into energy, and only a very small part of such wastes is employed for production of phytotherapeutic products. However, it is seldom considered that such biomasses, being materials of a high environmental impact and high disposal costs, could be considered as new low-cost starting materials for the production of biological products having a high added value, for agro-food industry, phytotherapeutic industry and cosmetic industry.

From an updated market survey it appears that the commercial olive leaf extracts available on the market are not many, and that the existing ones have a low titer in oleuropein (a bitter-tasted glucoside which is the main constituent of the leaves, but is also present in other parts of the plant, such as fruits, bark, roots) which varies from a minimum of 5% to a maximum of 12%. The main compounds identified in the leaves are secoiridoids (mainly oleuropein and ligustaloside), which have also been found in low amounts, in the form of aglycones, in the oil and in the waste products, hydroxytyrosol, several phenolic acids, phenylpropanoids such as verbascoside and derivatives thereof, as well as flavonoids, mainly glycosides of luteolin and apigenin, but also quercetin and chrysoeriol. By virtue of such chemical composition, olive leaves are used since long time ago to obtain an antihypertensive, hypocholesterolemizing and hypoglycemizing action.

The olive leaves have been used by popular medicine to fight fever and other diseases, including malaria. Several studies evidenced that extracts of olive leaves lower the blood flow in the coronary arteries. More recently, Benavente-Garcia et al. (Benavente-Garcia 0., Castillo J., Lorente J., Ortuño A., Del Rio J. A. Antioxidant activity of phenolics extracted from Olea europaea L. leaves. Food Chemistry, Vol. 68(4), March 2000, pp 457-462) showed a sequence as concerns the radical-scavenging activity of **polyphenols** present in the olive leaves, and of the extract itself: rutin> cathechin≈luteolin> olive leaves extract≈ hydroxytyrosol> verbascoside> oleuropein> luteolin-7-glucoside> apigenin-7-glucoside> tyrosol. The most active flavonoids have an activity at least 2,5 times higher than vitamins C and E.

Actually, the different compounds of vegetal origin are able to express different biological activities (e.g., antioxidant, radical scavenging, antimicrobial activity) and, in many case, the single molecule is less active than the mixture of compounds itself, this suggesting a synergic action between the same. In other words, it is not infrequent that crude extracts of a vegetal origin show a higher activity in comparison with the activity detectable when the single compounds of the extract are used alone. To this regard, the radical-scavenging activity of vegetal extracts is measured by *in vitro* tests with the aid of the stable DPPH radical (diphenyl-picryl-hydrazyl radical), which simulates the activity of such extracts towards the endogenous oxygenated radicals, such as hydroxide and superoxyde (Baratto M. C., Tattini M., Galardi C., Pinelli P., Romani A., Visioli F., Basosi R., Pogni R., "Antioxidant activity of galloyl quinic derivatives isolated from P. lentiscus leaves", Free Radical Res., 2003, 37(4), 405-412). By using the DPPH radical it is possible to both study the dampening kinetics of the radical and calculate the EC₅₀, i.e. the extract concentration which will suppress 50% of the radical itself (Heimler D., Vignolini P., Dini M., Vincieri F.F., Romani A. Antiradical activity and polyphenol composition of local Brassicaceae edible varieties. 2006, Food Chem, 99, 464-469; Romani A, Lapucci C, Cantini C, leri F, Mulinacci N, Visioli F. Evolution of Minor Polar Compounds and Antioxidant Capacity during Storage of Bottled Extra Virgin Olive Oil. J. Agric. Food Chem. 2007, 55, 1315-1320).

It is known that **hydroxytyrosol** and **oleuropein aglycone** are potent antioxidant and cardioprotective agents. Oleuropein shows a coronary-dilating, hypoglycemic and anticholesterolemic activity and, similarly to hydroxytyrosol, it delays oxidation of LDLs. Hydroxytyrosol has been shown to reduce the gene expression of iNOS and COX-2 cell lines, thus preventing the activation of the transcription factors NF-_{K}B, STAT-1a and IRF-1 (maiuri MC, Di Stefano D, Di Meglio P, Irace C, Savarese M, Sacchi R, Cinelli MP, Carnuccio R. Hydroxytyrosol, a phenolic compound from virgin olive oil, prevents macrophage activation. Naunyn-Schmiedeberg's Arch Pharmacol 2005; 371:457). NF-_{K}B is known to play an important role in atherosclerosis (De Winther MPJ, Kanters E, Kraal G, Hofker MH. Nuclear factor kB signaling in atherogenesis. Arterioscler Thromb Vasc Biol 2005; 25: 904). Some *ex-viv0* studies on healthy volunteers showed that, differently from meals based on butter, the olive oil consumption does not induce the activation of NF-_{K}B in monocytes, and this may suggest a possible anti-inflammatory effect of olive oil (Bellido C, Lopez-Miranda J, Blanco-Colio LM, Perez-Martinez P, Muriana FJ, Martin-Ventura JL, Marin C, Gomez P, Fuentes F, Egido J, Perez-imenez F. Butter and walnuts, but not olive oil, elicit postprandial activation of nuclear transcription factor kB in peripheral blood mononuclear cells from healthy men. Am J Clin Nutr. 2004; 80:1487).

The scientific literature comprises several examples of studies relevant to the biological activity of molecules with **phenylpropanoid** structure, in particular concerning their antioxidant, anti-inflammatory, antiviral and antimycotic activity (Daels-Rakotoarison DA, Seidel V, Gressier., Brunet C, Tillequin F, Bailleul F, Luyckx M, Dine T, Cazin M, Cazin JC. Neurosedative and antioxidant activities of phenylpropanoids from Ballota nigra. Arzneimittelforschung 2000; 50:16; Deepak M, Handa SS. Antiinflammatory activity and chemical composition of extracts of Verbena officinalis. Phytother. Res. 2000; 14:463). Most of such studies are mainly focused on evaluating the effects of **verbascoside** or acteoside, one of the phenylpropanoids most studied at present. Verbascoside also evidenced an *in vitro* immunomodulating activity; in particular it increases the chemotactic activity of neutrophils. An opposed effect has been observed on increasing the dose, probably due to a suppression of the killing activity of neutrophylic lymphocytes (Akbay P, Calis l, Undeger U, Basaran N, Basaran AA. In vitro immunomodulatory activity of verbascoside from Nepeta ucrainica L. Phytother. Res. 2002; 16: 593).

Another study (Sheng GQ, Zhang JR, Pu XP, Ma J , Li CL. Protective effect of verbascoside on 1-methyl-4-phenlpyridine ion-induced neurotoxicity in PC12 cells. Eur. J. Pharmacol. 2002; 451:119) showed, in addition, that acteoside has a protecting activity towards the neurotoxicity induced by 1-methyl-4-phenilpiridinium ion (MPP⁺), on neuronal cells PC12 cultures. This ion induces apoptosis by activating an enzyme, capsase-3, i.e. a DNA fragmentation factor, and causes serious oxidative stresses on the cells. Acteoside reduced the cell mortality with a dose-depending effect, thanks to its radical scavenger ability, thus reducing the oxidative stress and preventing, as a consequence, the capsase-3 activation resulting in apoptosis. The authors suggest that the use of such molecules could represent a useful therapeutic strategy in the treatment of neuronal degeneration induced by oxidative stress, such as in Parkinson disease.

An antineoplastic activity of verbascoside and its isomer, isoacteoside, has been reported as well. From *in vivo* tests carried out on murine leukemic cells P-388, the two phenylpropanoids have shown a cytotoxic activity at a concentration ED₅₀ of 10 µg/mL for isoverbascoside, and 26 µg/mL for verbascoside (Pettit GR, Numata A, Takemura T, Ode RH. Narula AS, Schmidt JM, Cragg GM, Pase CP. Antineoplastic agents, 107. Isolation of acteoside and isoacteoside from Castilleja linariaefolia. J Nat Prod. 1990; 53:456).

Another quite studied class of compounds in the olive oil is the class of **lignanes,** acetoxypinoresinol and pinoresinol. Also lignanes present an antioxidant activity, yet lower than that of the previously mentioned molecules (Nenadis N, Wang, L.F.; Tsimidou, M.Z.; Zhang, H.Y. Radical scavenging potential of phenolic compounds encountered in O. europaea products as indicated by calculation of bond dissociation enthalpy and ionization potential values. J. Agric. Food Chem. 2005; 53: 295). The existing structural similarity between lignanes, estradiol and the synthetic anti-estrogen tamoxifen suggests that lignanes exert an anti-tumoral action, in part as a result of anti-estrogenic effects. Actually, lignanes appear to inhibit the estrogen synthesis by the placenta and adipocytes; in addition, they inihibit the proliferation of human cells MCF-7 from breast carcinoma and stimulate the synthesis of estradiol binding globulin, thereby resulting in lowering of the free sexual hormone (Owen RW, Giacosa A., Hull We., Haubner R., Spiegelhalder B., Bartsch H., "The antioxidant/anticancer potential of phenolic compounds isolated from olive oil", Eur J Cancer, 2000 Jun, 36:1235).

A further polyphenol subclass (more specifically, a flavonoid subclass) of a particular biological interest, specifically present in the pulps of pigmented olives, is the class of **anthocyanoside** compounds (or anthocyanines), consisting, in particular, of glycoside derivatives of cyanidine, the most abundant of them being cyanidine-3-O-rutinoside (Romani A, Mulinacci N, Pinelli P, Vincieri FF, Cimato A. Polyphenolic content in five Tuscany cultivars of Olea europaea L. J.Agric.Food Chem 1999; 47(3):964-967). Anthocyanosides are pigments specifically present in the skin of grapes and small fruits, marketed as standardized extracts of berries or of marc residues, and widely studied in view of their biological activity. The term **anthocyanidines,** referred to the class of the corresponding non-glucosylated compounds (cyanidine being one of the major representatives thereof), has been created to designate the substances responsible for the color of flowers, and is relevant to a group of water-soluble pigments responsible for the colors red, pink, violet and blue of most flowers and fruits.

The drugs containing anthocyanidines were used for the treatment of the symptomatology connected with capillary fragility. Such compounds also show a high antioxidant power and are capable of protecting cells from oxidative damages caused by free radicals. Cyanidine and its glucoside cyanidine 3-0-beta-D-glucoside show protective effects on DNA and a significant inhibitory activity against oxidation by xanthine. A dose-depending *radical-scavenging* activity has also been shown, detectable at pharmacological doses.

Considering the commercial products based on olive leaf extracts, the crude product referred to as "Olive 6% dry hydroalcoholic extract" (CAS No. 84012-27-1) represents an example of what is presently available on the market. From the relevant technical data sheet it appears that it is a dry hydroalcoholic extract (ethanol/water) of *Olea europaea* L. leaves, the excipient of which is maltodextrin, and the active constituents of which, as declared, are as follows: "oleuropeosides (oleuropein and verbascoside), flavones (luteolin-7-glucoside, apigenin-7-glucoside diosmetin-7-glucoside, luteolin and diosmetin), flavonoids (rutin), flavan-3-ols (catechin), phenols substituted (tyrosol, hydroxytyrosol)". The same technical data sheet lists the following physiological and health-protecting purposes for the product: "hypotensive, hypoglycemizing, is used in hypertension and as an adjuvant in diabetes forms, antioxidant". The relative amounts of the various components mentioned are not specified.

As already noted, none of t he present commercial products obtained from *Olea europaea* leaves has an oleuropein content over 12% and, in any case, the concentration of oleuropein is not individually shown, in that what is declared is generally the overall concentration of the active principals present in the extract.

Several documents having as an object the production of olive leaves extracts are present In the patent literature, such as, for instance, the patent US5714150(Nachman), which discloses a method for preparing an olive leaves extract by macerating them in alcohol/water mixture at least 25% by eight in alcohol, at a temperature of from 20°C to 88°C, for a period of time of at least 4 hours. The limit of 88°C is due to the fact that oleuropein has a decomposition temperature of 90°C. In order to achieve a good extraction, the process has to be repeated at least twice with a fresh hydroalcoholic mixture, and upon joining the three extracts a concentrate extract is obtained by distillation under vacuum, having 30-40% by weight of solids. The dry product obtained by spray-drying the concentrate would contain, in turn, 30-40% by weight of oleuropein.

In a similar way, the patent application US2003/0017217 (Quintanilla Almagro et al.) discloses a process for producing *Olea europaea* leaves extracts wherein the starting material (olive leaves) is dried before the extraction at a temperature not above 35°C and away from direct light, in order not to damage the active principles contained therein. The dried leaves are then left to macerate in a hydroalcoholic mixture at temperatures below 20°C. The liquid extract affords, after concentration under vacuum and freeze-drying, and olive extract in powder with a yield of about 25%, containing about of 18% of oleuropein and about 5% of flavonoids.

Also the European patent application EP17952101A1(Mediterranean Agronomic Institute of Chania) concerns a method for extracting oleuropein from previously dried olive leaves, wherein the drying occurs away from direct light and at room temperature. Then, the leaves are ground and subjected to extraction with a weakly basic solution (pH=8) for 3-6 hours at room temperature, or for a shorter time if the temperature is 35-50°C. The filtered extraction residue undergoes a further extraction with organic solvent and the organic phase, which extracted most of the oleuropein, is distilled with a suitable dehydrating agent. The organic solvent is evaporated under vacuum, obtaining a dense liquid with 40-50% of oleuropein. Further purification by flash chromatography on silica gel column may lead to the production of oleuropein in the form of an amorphous powder, with a titer of 95-98%.

All of the procedures described above are specifically focused on the extraction of oleuropein alone from the *Olea europaea* leaves, in view of the production of herbal extracts or for products for use in cosmetics.

An exploitation of the olive tree leaves for the recovery therefrom of various substances of high added value, finalized to exploiting an agricultural residue rather than to the production of a specific extract, is disclosed in the international patent application publ. No. W02005/075614 (Consejo Superior de Investigaciones Científicas and Universidad Autonoma de Madrid). The olive leaves, both from pruning residues and from olive mill wastes, are first treated by extraction with an organic solvent, in particular hexane or ethanol. The document suggests to dry the leaves in air under forced ventilation at 30-40°C and chop them if hexane is used for the extraction, while it suggests to leave them intact without drying them if ethanol is used. After the extraction the product is filtered and concentrated under vacuum, and the crude extract is subjected to fractionation with supercritical CO₂. Three fractions are obtained in total, with different contents of the various components of the olive tree leaves.

Also the process disclosed in the international patent application publ. No. W02007/013032 (De Magalhães Nunes Da Ponte et al.), is based on a separation by extraction with supercritical CO₂. The document describes a procedure for the recovery of a concentrate rich in hydroxytyrosol from the residues of the oil production industry. In this case the pruning waste is not considered, and the residues to be recovered are, in practice, the olive mill wastes, i.e., vegetation waters (VW), pomace and also leaves, but in a very small relative amount. For that reason, actually, the product to be recovered on which the process is mainly focused is not oleuropein but hydroxytyrosol, which is mostly found in the olive mill wastes.

According to the proposed process, after the conventional extraction with hydroalcoholic solvent the resulting liquid fraction undergoes extraction with supercritical CO₂ or, as an alternative, a membrane nanofiltration operation (NF). In the first case the fraction enriched in hydroxytyrosol is obtained in the extract, while in the second case the enriched fraction is obtained in the NF permeate. The thus obtained fraction is fed to a reverse osmosis operation (RO), which gives in the RO retentate a hydroxytyrosol-enriched product.

A separation process based on membrane technologies, similarly to the process described above, but specifically directed to the recovery of various useful components from vegetation waters, has already been proposed, *inter alia,* by some of the authors of the present invention in the international patent application publ. No. W02005/123603 (ENEA - Ente per le Nuove Tecnologie, l'Energia e l'Ambiente and Verdiana srl). In such process, an initial treatment aiming at maximizing the contents of commercially useful polyphenols (such as hydroxytyrosol) with respect to any oleuropein possibly still present in the VW. The pretreatment consists of acidifying the freshly produced VW to a pH of about 3-4.5, followed by an enzymatic hydrolysis. After separation by centrifugation of the liquid product thus treated, a series of tangential filtration operations are carried out one after another, obtaining from the various retentates some polyphenol fractions having different grades of purification and from the reverse osmosis permeate a purified water, useful for the production of beverages.

A further example of recovery of the hydroxytyrosol component from the oil industry waste, where, however, not only the olive mill waste (specifically, pomace) is employed, but also a certain amount of green olives, in order to obtain a product particularly rich in hydroxytyrosol, is disclosed by the international patent application publ. No. W02008/090460 (Probelte Pharma S.A.). The document proposes a first treatment of acid hydrolysis at a temperature above the reflux temperature of the starting material (i.e. pomace and green olives pulps), followed by a clarification of the resulting product (for instance, by filtration), followed in turn by a treatment on a ion exchange chromatographic resin. The product adsorbed on such column, after elution, can be in turn fed to a second chromatographic column, loaded with a non-ionic adsorbent resin. The product adsorbed on the latter resin, after elution, is further concentrated in hydroxytyrosol, if necessary, by means of a membrane tangential filtration treatment, specifically reverse osmosis, the retentate of the latter being the desired product.

In the light of such prior art, an object of the present invention is, therefore, to provide a productive system which allows to advantageously reuse the residues of the olive growing and oil production sectors, while taking care of improving the value of the huge amounts of biomass being originated not only by the oil milling industry, but from the whole sector of *Olea europaea* growing and productive exploitation. The said biomass should be converted, thus, into a wide range of high added-value products for the food and pharmaceutical industry. Besides being suited to the production of diversified products, the proposed method is to be such as to respect the environment and the ecosystem.

To that aim a process has been devised, according to the present invention, which affords the production of purified fractions, in powder or in the form of concentrated solutions, having different compositions in active principles of high biological value, starting from more different matrices. Such matrices comprise both the olive tree pruning waste (olive leaves and twigs, either fresh or previously dried) and the solid or semisolid olive mill residues (wet pomace from the two-phase extraction process, leaves and twigs), as well as destoned olive pulps added on purpose, specifically from pigmented olives. Such fractions may contain different subclasses of antioxidants, purified and standardized, some of which are not yet present on the market, such as, e.g., lignanes and anthocyanosides, which can be employed in the food industry (e.g. production of nutraceutics and novel food) and, in addition, in the phytotherapeutic and pharmaceutical sectors. Said fractions may undergo further enrichment up to a high titer, or they may undergo a suitable separation to yield isolated compounds.

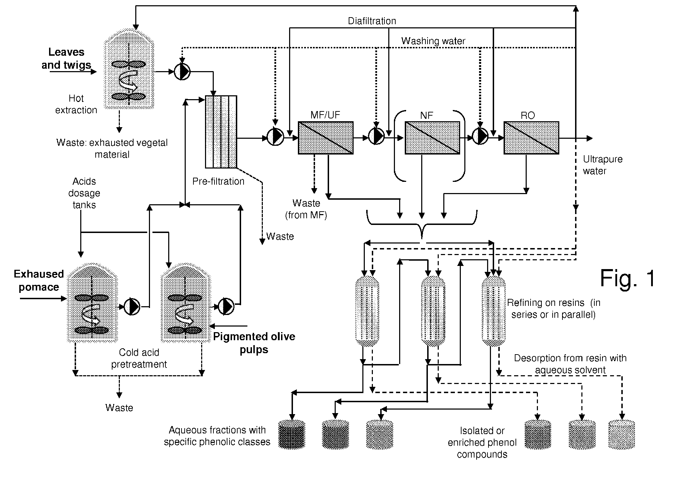

The proposed process (schematically shown in the enclosed **Figure 1**, which will be discussed in the following) is based on the integration of suitable preliminary liquid-solid extraction operations, carried out on the starting matrix with simultaneous heating or at room temperature according to the starting matrix, followed by two or more consecutive tangential membrane filtration operations, carried out on the clarified liquid product coming from the extraction and, if required, further followed by a series of operations of adsorption and desorption of the active compounds contained in the various retentates of the tangential filtration operations, carried out with specific resins. The latter step allows to increase the titer of the bioactive components having a commercial interest, and to obtain purified active principles belonging to different phenolic subclasses, such as secoiridoids, hydroxycinnamic acids, glucosylated flavones and anthocyanosides.

In particular, for a starting matrix consisting of olive leaves and twigs, either fresh or dry, the process according to the invention includes an extraction operation, with heating or by means of a pneumatic pressure extraction, followed (after filtration, if necessary) by two or more tangential membrane filtration operations in series, in particular a microfiltration (MF) or an ultrafiltration (UF), followed by reverse osmosis (RO) carried out on the MF or UF permeate, and preferably a series of operations of adsorption and desorption of the active compounds contained in the RO retentate by means of specific resins. For starting materials consisting of exhausted pomace from the two-phase extraction process, possibly diluted, if dry, with vegetation waters, as well as for the pigmented olives pulps, the process includes, after a preliminary acidification, an extraction at room temperature (cold extraction) of the polyphenol compounds contained in each matrix, followed, after filtration, by a sequence of tangential membrane filtration operations, similar to that used for the starting materials consisting of leaves and twigs, and also followed, if required, by a further purification on specific adsorption resins.

The products thus obtained can be marketed either as concentrated solutions and as powders having a standardized titer. In the second case the RO concentrate (retentate) and the product desorbed from the resins thus obtained are dried or spray-dried to give stable powders rich in antioxidants of biomedical interest belonging to different polyphenol classes. The active compounds obtainable through the process of the invention are mainly flavonoids (glucosides of luteolin and quercetin), polyphenols derivatives (hydroxytyrosol and hydroxytyrosol glucoside), secoiridoids (oleuropein and ligustaloside), verbascoside, caffeic acid derivatives and pigments of an anthocyanoside nature.

The RO permeate is partly re-used to perform diafiltration operations on the membranes, and partly is recirculated to the first operation of extraction of the starting biomass.

When required, the proposed process also includes an intermediate nanofiltration (NF) step, to be applied to the MF or UF permeate in order to diversify the refined products obtainable from the combination of the various operations. For instance, in the event that the active principle of interest contained in the olive extract is oleuropein, the NF applied to the MF permeate obtained from the extract of fresh leaves and twigs allows to concentrate this component, besides others having molecular weight below 200 kDa, in the following RO retentate. Oleuropein may be obtained in this way in a refined form, with titers higher than those obtainable from the known extraction processes.

According to what proposed by the method of the present invention, the process starting from leaves and twigs as a starting material may be more specifically aimed at obtaining phenolic compounds, such as hydroxytyrosol, or in the alternative at obtaining high yields of oleuropein, by simply suitably selecting the starting material to be fed to the process. As it is shown by the experimental data presented in the following, by using in the initial extraction fresh olive leaves instead of dry leaves the extract obtained is remarkably richer in oleuropein, while the previous drying dramatically reduces the content of such component in the extract, reducing the concentration of total polyphenols and enhancing the production of extracts with high contents of flavonoid and hydroxycinnamic derivatives.

On the other hand, the introduction of an amount of starting material consisting of pigmented olive pulps allows to obtain, among the final products, also components based on anthocyanoside pigments. These are normally not present in the products that may be recovered from the olive mill residues, nor from the pruning waste of the olive cultivations.

Therefore, the present invention specifically provides a process for producing concentrated actives and/or refined extracts from tissues and by-products of *Olea europaea* comprising a first section operating starting from leaves and twigs, a second section operating starting from wet pomace produced by two-phase oil extraction processes and a third section operating starting from destoned pigmented olive pulps, wherein the said first section comprises, in sequence, the following operations:
a) providing a first starting material consisting of olive leaves and twigs, suitably pre-chopped if necessary, with a predetermined moisture content;
b) extracting active components from the solid matrix of the said first starting material with an aqueous or hydroalcoholic solvent;
c) treating the liquid phase resulting from the previous operation by tangential microfiltration (MF) or tangential ultrafiltration (UF), to give a retentate phase and a permeate phase;
d) treating the permeate resulting from the previous operation by reverse osmosis (RO), to give a retentate phase enriched in polyphenol active principles and a permeate phase consisting of demineralized water;
the wet solid or semisolid vegetal residue obtained from said extraction b) and from the possible phase of MF retentate from the operation c) being reusable as animal feedstock or for the production of fertilizers or energy, and the possible phase of UF retentate coming from the operation c) being enriched as well in polyphenol active principles,
and wherein said second and third sections each comprise at least a cold extraction treatment from the corresponding starting material, carried out in an acid aqueous solvent at a pH comprised between 1 and 6.

As pointed out before, the starting material provided in step a) may either consist of olive tree pruning waste or of solid oil mill residues mainly consisting of leaves and twigs. In particular, the leaves may be fresh, with a moisture content roughly similar to that of just trimmed olive leaves, or the vegetal material may previously undergo drying, in particular by treating it in forced heated air.

In order to optimize a drying in forced heated air, in the frame of the experimentation carried out in connection with the invention, the fresh material has been treated at 40°C for 24 hours, the moisture loss has been evaluated and the qualitative and quantitative features of the polyphenol contents of both fresh leaves and dried leaves have been evaluated. As it appears from the Examples shown in the following **(Table 2),** the fresh leaves should be used by preference for obtaining extracts standardized both in total polyphenols and in oleuropein, which are also useful due to the relevant hypotensive properties. On the other hand, the dried leaves are the most suitable material for obtaining extract with high concentration of flavonoids and hydroxycinnamic derivatives, which may be used as antioxidants and for the prevention of cardiovascular diseases.

A further parameter considered was the size of the vegetal material, which appears to be critical to the extraction yield. Depending either on the extraction method employed in the operation b) of the process, either on the solvent used and on the kind of products desired, it is possible to define the opportunity or less to carry out a preliminary crushing or chopping of the starting vegetal material.

According to some specific embodiments of the invention, the cited second section of the process proposed comprises, in sequence, the following operations:
A) providing a starting material consisting of wet pomace produced by two-phase oil extraction processes, diluted with water or with vegetation water in an amount sufficient to obtain a semisolid consistence, and acidifying to a pH comprised between 2.5 and 4, in order to favor the extraction of the bio-components and stabilize the chemical environment conditions;
B) cold extraction, with an aqueous solvent, of active components from the solid matrix of said second starting material;
C) treating the liquid phase resulting from the previous operation by tangential microfiltration (MF) or tangential ultrafiltration (UF), to give a retentate phase and a permeate phase;
D) treating the permeate resulting from the previous operation by reverse osmosis (RO), to give a retentate phase enriched in polyphenol active principles and a permeate phase consisting of demineralized water;
the wet solid or semisolid vegetal residue obtained from said extraction B) and from the possible phase of MF retentate from the operation C) being reusable in the same way as the residual fractions originated from the main section of the process, and the possible phase of UF retentate coming from the operation C) being enriched as well in polyphenol active principles.

Similarly, also the third section of the process proposed according to the invention, directed to treat the pigmented olive pulps after destoning of the same, may comprise a series of operations of the same kind as those carried out in the main section of the process. Specifically, said section comprises, in sequence the following operations:
i) providing a starting material consisting of destoned pigmented olive pulps, and acidifying to a pH comprised between 2.5 and 4, in order to stabilize the anthocyanoside components and cyanidine derivatives;
ii) cold extraction, with an aqueous solvent, of active components from the solid matrix of said starting material, particularly rich in anthocyanosides;
iii) treating the liquid phase resulting from the previous operation by tangential microfiltration (MF) or tangential ultrafiltration (UF), to give a retentate phase and a permeate phase;
iv) treating the permeate resulting from the previous operation by reverse osmosis (RO), to give a retentate phase enriched in anthocyanoside components, oleuropein and other polyphenol active principles, and a permeate phase consisting of demineralized water,
the residue obtained from said sequence of operations being reusable in the same way as the previous residual fractions, and said possible phase of UF retentate coming from the operation iii) being enriched as well in anthocyanoside components, oleuropein and other secoiridoids and polyphenol active principles.

According to some specific versions of the procedure proposed by the present invention, in all of the three sections of the process described, downstream of the phases c), C) and/or iii) of tangential microfiltration (MF) or tangential ultrafiltration (UF), there may be inserted, depending on the fractions desired in the product, the following further operation:
c1) treatment of the permeate resulting from the previous operation by tangential nanofiltration (NF), to give a retentate phase and a permeate phase, the permeate being fed to said phase d), D) or iv) of reverse osmosis.

As already noted, the opportunity to insert also the nanofiltration before the last treatment of tangential membrane filtration (RO) mainly depends on the kind of product that should be obtained by preference from the series of separations included in the process. A NF membrane with a molecular cut-off lower than that of the MF and UF membranes allows to separate a quite limited range of sizes in the relevant retentate, corresponding, in particular, to the molecular size of oleuropein, while it allows the smaller molecules to pass through it, towards the following RO operation.

Considering in detail the scheme of the process proposed according to the invention, the extraction of the polyphenol compounds from the biomass of the first matrix (leaves and twigs) may take place according to two different procedures:
- hot extraction procedure;
- pneumatic pressure extraction procedure.

According to the first alternative, the operation b) of extraction with an aqueous or hydroalcoholic solvent is more specifically an operation of hot infusion. The vegetal material to be treated is placed in the infusion bath in a weight/volume ratio of leaves-twigs to water (or hydroalcoholic solution) comprised between 10% and 35%, at temperatures comprised between 60°C and 95°C, for periods of time comprised from 15 to 60 min..There follows an operation of:
b1) mechanical separation of the solid material obtained from the previous operation, by filtration,
said operations b) and b1) being repeated at least a second time in the event that a more thorough extraction of said active principles is desired. The filtrate obtained is fed to the following operation c) of tangential membrane separation.

Upon some preliminary studies carried out mainly on fresh olive leaves a preferred hot extraction protocol with distilled water has been defined. The following **Table 1** reports the preferred conditions for the extraction of polyphenols from olive leaves and twigs.

**Table 1**

| Preferred conditions for hot extraction of polyphenols from the biomass of the first matrix | |
|---|---|
| Biomass/water weight ratio (%) | 20-30 |
| Temperature of the bath (°C) | 70-80 |
| Extraction time (minutes) | 25-35 |
| Number of batches | 2 |
| Extracting liquid | osmotized water |
| Electrical conductivity (µS/cm) | 6-8 |

According to the cited protocol, the vegetal biomasses are soaked in osmotized water, obtained from a reverse osmosis process starting from tap water. This occurs in the start-up phase of the process, i.e. in the first batch of hot water extraction. In all further cycles of extraction according to the invention the water deriving from the reverse osmosis permeate is employed, so that no process water consumption is involved.

In the experimentation carried out the weight of the twigs was about 12% with respect to the olive leaves.

After the infusion, the organic mass of leaves and twigs was left to drain, the tap on the bottom of the boiler was closed and then demineralized fresh water was added in order to proceed to a second bath of aqueous extraction, which was carried out with the same working parameters of the first one.

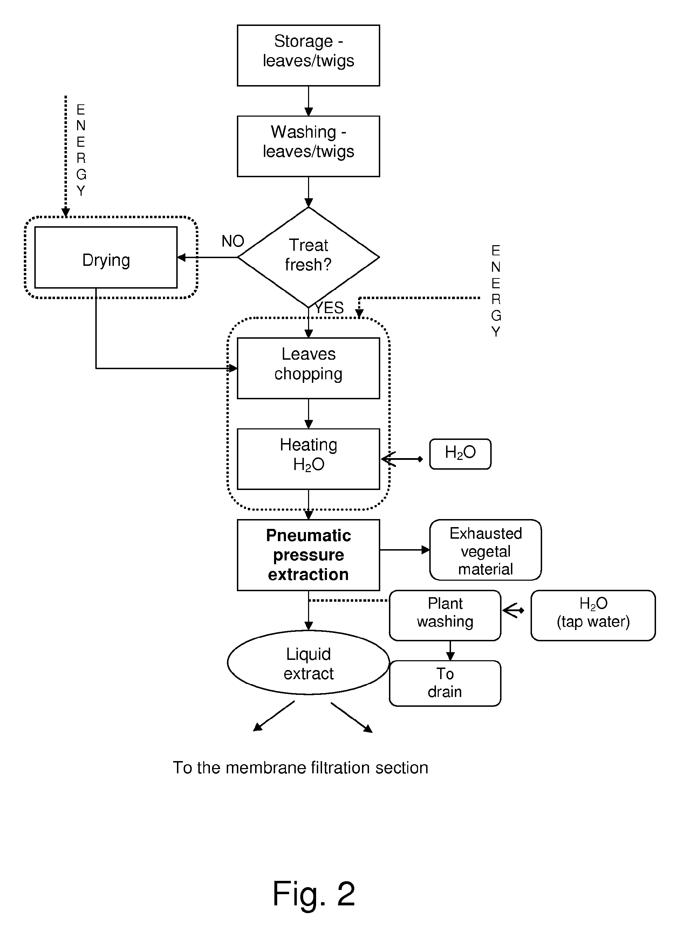

In the event that the pneumatic pressure extraction procedure is adopted, based on a solid-liquid extraction process (and schematically shown in the enclosed **Figure 2****)**, the said step b) consists of an operation which alternates a dynamic step, obtained under a programmed pressure, with a static step, wherein the extractable substance is transferred into the aqueous phase, and said operation thus affords a clear liquid extract, which is directly fed to the following operation c) of separation of the components by tangential membrane filtration.

In this case leaves and twigs from pruning, initially stored in suitable containers, are taken preferably in the ratio of 10-15% of the weight of the aqueous extract which is to be obtained in order to undergo the following washing step. Then the leaves may undergo drying or be sent fresh directly to the chopping step, depending on the kind of extract that it is desired to obtain.

In the case of drying, the leaves undergo a treatment based on subtraction of moisture from the vegetal matrices by means of forced circulation of heated air, reaching a temperature of about 60°C for 48h. Such treatment allows to preserve the quality and organoleptic characteristics of the material subjected to extraction, while offering the possibility of obtaining extract much more enriched in flavonoids with respect to oleuropein.

The material, fresh or dried, is then loaded into a hopper ant chopped through the action of pre-cut knives and counter-knives keyed on a rotating disc, and then the product is collected to be sent to the extraction phase. The chopping is necessary in order to maximize the extraction efficiency, in that it allows to increase the contact surface between the vegetal matrix and the solvent.

The aqueous phase wherein the extracted substance is transferred is heated simultaneously in a suitable container at controlled temperature, by means of a heating jacket with oil o another system for thermal control (e.g., 250 liters of water at 90°C for 5 hours). During the dynamic phase a forced percolation is generated with a recirculation of the extracting liquid and at the same time the formation of preferential channels and the oversaturation of the product are avoided. Said technology allows to increase the extraction productivity while leaving unaffected the quality and organoleptic properties of the active principles. Further, it affords the use of water alone or of mixtures of liquids, for instance water/ethanol, in case a co-extraction of other slightly soluble or liposoluble substances is necessary.

At the end of the cycle, with an automatic control of the pressure in the extraction chamber, the product is pressed in order to obtain the maximum extraction. The double action of the pistons, by pushing the solvent into the vegetal material, generates a movement of liquid in the extraction chamber:
- extraction fastness and limited management costs;
- production of clear and pre-filtered extracts;
- control of the treatment phases with microprocessors;
- simplicity of use, maintenance and clearing;
- maximum reliability and high yield of the plant;
- flexibility and constant repeatability of the various extractions;
- extraction at room temperature and thus with reduced energetic consumption;
- organoleptic characteristics of the extracted actives unaltered;
- minimal loss of solvent during the various steps.

Also in the case of pneumatic pressure extraction, starting from the biomass of olive leaves and twigs, the preferred weight/volume ratio of leaves and twigs fed to said operation b) to water or hydroalcoholic solution is comprised between 10% and 35%.

Considering the second and third sections of the process proposed according to the invention, which start, respectively, from a matrix consisting of pomace from the two-phase oil extraction process and from destoned pigmented olive pulps, as noted before, in both cases the operation of extraction of the substances of interest from the matrix is carried out at room temperature, and after such operation the following further operation is preferably carried out :
b1) mechanical separation of the vegetal solid material obtained from the previous operation, by filtration,
the filtrate obtained being fed to the following operation C) or iii) of separation of the components by tangential membrane filtration.

With specific reference to the case of a starting material consisting of olive leaves and twigs, in the experimentation conected to the present invention, after an extraction carried out according to the first alternative mentioned above, i.e. with a hot extraction process, the aqueous solutions that had extracted the active principles from the matrices considered, i.e. fresh leaves, dried leaves and olive twigs, were processed with different membrane techniques. This, in order to correlate the process parameters and the kind of membrane used with the chemical composition of the final products obtained in liquid form (RO concentrates), and with the powder obtained from the same solutions by drying upon addition of maltodextrins.

The MF and UF techniques are carried out, in the process according to the invention, in order to purify the aqueous extract from suspended and dissolved substances different from the polyphenol-based actives. Such purification step is always carried out for all of the matrices treated (fresh and dried olive leaves and twigs). The RO and NF techniques are applied on the permeate solutions coming from the MF or UF treatment.

According to some preferred versions of the process of the invention, the operation c) of tangential microfiltration (MF) is carried out with polymeric membranes, preferably made of polysulfone, regenerated cellulose acetate or nylon, in spiral-wound form, or with ceramic membranes consisting of a tubular ceramic monolith, made of alumina with an internal coating of zirconia or of titanium oxide.

The molecular cut-off of the MF membranes is preferably comprised in the range 0.10-3.0 µm.

Similar solutions are proposed, according to the invention, for the operations of microfiltration C) and iii), respectively carried out in the section treating wet pomace from two-phase oil extraction processes and in the section treating pigmented olive pulps.

As concerns the UF operation, to be carried out as an alternative to MF, in all of the three cases (leaves and twigs, pomace from two-phase process and pigmented olive pulps) said operations c), C) and iii) of tangential ultrafiltration are preferably carried out with polymer membranes with a molecular cut-off comprised between 500 Da and 50 kDa, in spiral-shaped form.

The RO permeate, consisting of a demineralized water, is in part reused, preferably for the preparation of the extraction bath, i.e. for the production of the extract, thus avoiding the consumption of tap water or water from well. A further amount of RO permeate is fed to the various tangential filtration apparatuses for the diafiltration.

According to the integrated process proposed, actually, in each of the treatment sections using membrane separation techniques, downstream said operations c), C) and iii) of tangential microfiltration or ultrafiltration, a diafiltration is preferably carried out, feeding the membrane with demineralized water obtained from the operation d), D) or iv) of reverse osmosis, which is added to the MF retentate or to the UF retentate.

The reverse osmosis is always employed in the process according to the invention in order to concentrate the actives in the concentrated liquid effluent, but attention has also been given to the chemical composition of the permeate, in view of the interest that this fraction may involve in the field of functional beverages.

Preferably, the operations d), D) or iv) of reverse osmosis are carried out with low or high saline rejection spiral-shaped polymeric membranes, at a working pressure comprised between 7 and 35 bar (preferably 26 bar), in the temperature range from 5°C to 60°C, with feed rates of 0.5-2 m²/h for filtrating modules of 4 inches in diameter x 40 inches in length (10.16 cm x 101.6 cm). Preferably, said spiral-shaped RO polymeric membranes have a net spacer of a thickness comprised between 20 and 38 mil (0.51-0.97 mm), and the sizes of the filtering modules are 4" in diameter x 40" in length, 6" in diameter x in length (15.24 cm x 101.6 cm) and 8" in diameter x 40" in length (20.32 cm x 101.6 cm).

According to a specific embodiment of the invention, the optional operation c1) of tangential nanofiltration is carried out with a polymer membrane having molecular cut-off comprised between 150 and 250 Da, spiral-shaped. In an experimental example the NF was employed on the fresh leaves extract with the aim of concentrating oleuropein and all of the compounds with molecular weight below 200 kDa, again with the reverse osmosis technique.

In order to optimize the processes of enrichment and isolation of the biocomponents, according to some preferred embodiments of the present invention, the membrane fractionation technique is integrated with resin adsorption and desorption techniques. In this case, the phases obtained as the retentate from said treatments d), D) and iv) of reverse osmosis, those obtained as the retentate from the treatments c), C) and iii) of ultrafiltration, when present, and those obtained as the retentate from the treatments c1) of nanofiltration, when present, may, therefore, be subjected to treatments of adsorption on chromatographic resins to give corresponding enriched and refined products.

According to some specifically preferred embodiments proposed for the refining treatment on resins, the following resins are preferably employed: MN 202, a ionic exchange type resin consisting of a crosslinked copolimer styrene/divinylbenzene, XAD4, a hydrophobic polyaromatic resin with dipolar moment 0.3, and XAD7, a resin of the kind of acrylic ester, with a dipolar moment 1.8 (Rohm and Haas Co.).

After the treatment on resin, the enriched and refined polyphenol products obtained by desorption from the resins and/or the aqueous fractions containing specific polyphenol classes not adsorbed on the resins, that are obtained from the bottom of the corresponding columns, are dried by spray-drying or by freeze-drying. According to some specific embodiments of the invention, the final refined products are obtained by spray-drying, after a previous addition of maltodextrines or dextrans.

It is to be noted that the production of the extracts from residues and tissues of the olive plant according to the method of the present invention I based on a refining process which employs separation techniques by means of membranes, which are considered to be safe, reliable and innovative (Best Available Technologies). Such techniques do not introduce contaminating substance, operate at room temperature and therefore do not thermally damage the matrix. They are perfectly sterilizable, and therefore safe from the point of view of their possible microbiological contamination. The techniques in question are widely tested also in the most delicate pharmaceutical preparations.

An analysis using the Life Cycle Assessment (LCA) method according to the ISO 140001 normative has been applied to the process proposed to the invention and to all of its steps, in order to evaluate the environmental impact associated to the processes considered and to the products obtained from the various matrices, for identifying and quantifying of mass and energy consumption and of the emission released in air, water and soil during all of the steps of the life cycle of technologies. A systematic approach has been adopted, considering for each phase the production of materials for the plant construction, the transportation, the energies, the water consumption and the end of life scenarios for each of them. The target was to individuate and evaluate the opportunities to improve the technologies from an environmental and energetic standpoint, and at the same time to make the products obtainable traceable, in the light of an improvement of the whole production chain of the sectors of interest (i.e., cosmetics, phytotherapeutics and food). The potentialities of the instrument have been fully exploited, by using a wide variety of available indicators, from the consequences on the human health to the air pollution, to the measure of the biodiversity. To that issue, it had been considered important to give evidence to the aspect of the improvement of the territory resources, which is a peculiarity of the invention.

The present invention also involved the fine tuning of analytical methods for characterizing and quantifying the active compounds obtained in the various fractions obtained, of tests for the *in vitro* evaluation of the antioxidant and radical-scavenging activity of said fractions and the optimization of specific technical data sheets for the traceability and the certification of the qualitative and quantitative composition and of the biological effectiveness of the product.

The specific technical features of the invention, as well as the advantages of the same and the relevant operating modalities, will appear more evident with reference to detailed description presented for merely illustrative purposes in the following, together with the results of the experimentation carried out on the same. The schemes of the proposed process and some experimental results are also shown in the attached drawings, wherein:
**Figure 1** shows a block diagram of an embodiment of the process for the production of refined actives starting from tissues and by-products of *Olea europaea* proposed according to the invention;
**Figure 2** shows a flow-chart of a pneumatic pressure extraction process from leaves and twigs of *Olea europaea* employable in place of the hot extraction in the process proposed according to the invention;
**Figure 3** shows the HPLC chromatographic profile, acquired at 240 and 330 nm, of a hot extract of fresh olive leaves and twigs resulting from the operation b) of the process according to the invention;
**Figure 4** shows the productivity curve, i.e. the trend over time of the permeate flow rate (L/m² h), in the UF and the UF/DF tests through the ultrafiltration membrane of Example 3;
**Figure 5** shows the productivity curve, i.e. the trend over time of the permeate flow rate (L/m² h), in the RO test through the reverse osmosis membrane of Example 3;
**Figures 6** and **7** show, respectively, the productivity curves (L/m² h), in the MF tests through the microfiltration membrane of Examples 4 and 5;
**Figure 8** shows the productivity curve (L/m² h) in the RO test through the reverse osmosis membrane of Example 5;
**Figure 9** shows the productivity curve (L/m² h) in the MF test through the microfiltration membrane of Example 6;
**Figure 10** shows the productivity curve (L/m² h) in the NF test through the nanofiltration membrane of Example 6;
**Figures 11** and **12** show the productivity curve (L/m² h) in the polymeric UF test through the 100 kDa ultrafiltration membrane, and that of the RO test through the reverse osmosis membrane of Example 7; and
**Figure 13** shows the HPLC chromatographic profile, acquired at 240 and 330 nm, of a hot extract of olive twigs resulting from the operation b) of the process according to the invention.

As it is shown in **Figure 1**, a specific embodiment of the process according to the invention comprises a section using, as a starting material, olive tree leaves and twigs (first matrix), either fresh or after suitable drying, that is to say with a predefined moisture content. Such choice, as already observed, is mainly depending on the kind of components which are desired in the final product.

In the embodiment shown in the figure, the starting material undergoes hot extraction, and the extract thus obtained is pre-filtered to give a clear liquid, which is fed to the section of treatment by tangential membrane filtration. In the event that a pneumatic pressure extraction is preferred to the hot extraction, the first two operations shown in Figure 1 can be replaced by the process schematically shown in the flow chart of **Figure 2****.** The latter does not require any pre-filtration of the extract. Figure 2 also shows, schematically, the procedure preliminary to extraction, affording a prefixed moisture content in the material (leaves and twigs) to be treated, and the chopping operation, which is not always needed when the hot extraction process is performed.

With reference to the scheme of Figure 1, the clear liquid is treated with a series of tangential filtration operations in a sequence, the first one of which is a microfiltration or an ultrafiltration, as it is shown more in detail in the following examples. There follows, in some cases, a nanofiltration on the permeate coming from the previous operation and, in all cases, the last membrane operation considered is a reverse osmosis. The latter produces, as the permeate, a high purity osmotized water, a part of which is recirculated as shown in the Figure, to the upstream operations.

The products (retentates) obtained from the UF and the NF, when present, and in all cases the RO retentate, contain precise fractions of the compound originally present in the olive leaves and twigs. The latter may be placed on the market as such or, preferably, they are treated in order to obtain refine fractions in the subsequent section of treatment on chromatographic resins. As shown in Figure 1, such refining phase is carried out, according to the invention, by a passage on suitable chosen chromatographic resins, in series and/or in parallel, thereby obtaining high purity adsorbed products, and liquid residues containing specific classes of phenolic compounds. The final products may be placed on the market as such, or they may be dried (not shown), generally together with a solid support such as maltodextrins, in order to be sold in the form of powders.

**Figure 1** also shows the other two sections in the proposed process, which work starting from pomace coming from the two-phase oil extraction process (second matrix) and from pigmented olive pulps (third matrix). In both cases, the part of the process which differs from the one illustrated above is the initial part for the extraction of starting material, which is carried out in both cases by a cold treatment and with the addition of an acid, in such dosages as to bring the pH to the desired value, not above 6. After the extraction, the pre-filtration of the extract and the subsequent treatments of tangential membrane filtration and refining on chromatographic resins are similar to those described above for the first matrix. Figure 1 shows, for the sake of clarity, only one production line, but the various apparatuses may be doubled or variously connected, to give an integrated plant wherein the various treatments may be performed according to the current needs, and specifically according to the characteristics of the material to be treated and of the final products desired.

### EXAMPLE 1

### Hot extraction from leaves and twigs (first matrix)

The fresh material consisting of olive tree leaves and twigs has been treated in hot forced circulation air at 40°C for 24 hours, so as to achieve dried samples having prefixed moisture contents. During the operation the loss of moisture and the qualitative and quantitative features of the polyphenolic contents of the fresh leaves have been evaluated at the various degrees of dryness.

Further, the various samples were subjected to hot extraction by infusion in water [phase b) of the process according to the invention], carrying out more tests with different percent amounts of leaves, both fresh or dried, with different water temperature and extraction times. The aqueous extracts obtained by the infusion have been analyzed by HPLC/DAD analysis in order to determine the titers of the single polyphenol compounds present in the extract.

Some of the obtained results for two different varieties of *Olea europaea* and, in both cases, for fresh and dried leaves, are reported in the following **Table 2**. In all cases the optimal extraction period was 60 min., while the w/v ratio of leaves to water considered to be the most suitable one was 15% in case of fresh leaves and 10% in case of dried leaves.

**TABLE 2**

| Analysis of the compounds present in aqueous extracts of olive leaves (HPLC/DAD) (mg/mL of aqueous extract) | | | | |
|---|---|---|---|---|
| | **Variety 1** | | **Variety 2** | |
| | **15% fresh leaves 60 min** | **10% dry leaves 60 min** | **15% fresh leaves 60 min** | **10% dry leaves 60 min** |
| | | | | |
| Hydroxytyrosol glycol | 102.68 | 125.21 | 0.00 | 81.03 |
| Hydroxytyrosol glucoside | 148.94 | 232.76 | 292.85 | 74.47 |
| Hydroxytyrosol | 63.80 | 28.50 | 73.70 | 3.91 |
| Hydroxytyrosol derivatives | 68.95 | 0.00 | 0.00 | 0.00 |
| Cinnamic acid derivatives | 0.00 | 0.00 | 0.00 | 382.46 |
| Oleoside (demethyl EA glucoside) | 127.56 | 72.12 | 157.88 | 6.22 |
| Demethyl EA diglucoside | 93.43 | 60.71 | 172.79 | 48.23 |
| Elenolic acid (EA) glucoside | 70.47 | 71.55 | 26.55 | 44.31 |
| Elenolic acid glucoside derivatives | 48.06 | 45.18 | 88.32 | 42.33 |
| Caffeic acid derivatives | 17.61 | 16.93 | 16.08 | 7.22 |
| P-cumaric acid derivatives | 3.96 | 3.28 | 1.80 | 0.00 |
| Verbascoside | 121.23 | 67.80 | 45.62 | 8.03 |
| Vitexin diglucoside | 0.00 | 0.00 | 0.00 | 4.99 |
| Luteolin diglucoside | 0.00 | 5.11 | 6.64 | 0.00 |
| Rutin | 9.40 | 29.12 | 13.76 | 3.32 |
| Luteolin 7-0-glucoside | 73.56 | 63.00 | 57.56 | 3.40 |
| Quercetin 3-0-glucoside | 4.89 | 6.02 | 5.81 | 2.43 |
| Apigenin 7-0-glucoside | 11.98 | 12.01 | 6.60 | 0,00 |
| Apigenin 7-O-rutinoside | 0.00 | 0.00 | 0.00 | 9.65 |
| Luteolin 4'-O-glucoside | 33.60 | 34.72 | 46.44 | 2.56 |
| Chrysoeriol 7-O-glucoside | 0.00 | 0.00 | 0.00 | 0.00 |
| Demethyloleuropein | 170.49 | 67.20 | 0.00 | 27.74 |
| 10 OH-oleuropein glucoside | 0.00 | 43.92 | 0.00 | 25.67 |
| Oleuropein | 1174.72 | 353.76 | 1749.48 | 16.52 |
| Oleuropein derivatives | 0.00 | 0.00 | 25.48 | 0.00 |
| Ligustaloside B | 129.76 | 66.08 | 189.84 | 0,00 |
| **TOTAL POLYPHENOLS** | **2475.09** | **1404.98** | **2977.18** | **794.49** |

As a result of the data reported in the table above, in the procedure according to the invention fresh leaves and twigs will preferably be used in case it is desired to achieve standardized extracts of both total polyphenols or oleuropein, useful as antioxidants and for their hypotensive properties. The dried leaves will be a preferred starting material in order to achieve extracts with high titer of flavonoids and hydroxycinnamic derivatives, which are used both as antioxidants and in cardiovascular diseases prevention.

### EXAMPLE 2

### Hot extraction from fresh leaves and twigs

The plant biomass, consisting of fresh pruning waste (leaves and twigs) from a Tuscanian cultivar of *Olea europea* L., has been put in infusion in osmotized water, obtained by reverse osmosis treatment of phase d) of the process according to the invention. The operative parameters adopted were the following:

| | |
|---|---|
| leaves+twigs/water weight ratio | 24% |
| extraction time | 30 min. |
| bath temperature | 75°C |
| batch number | 2 |
| extracting liquid | osmotized water. |

18 kg (dry weight) of fresh olive tree leaves and twigs has been used, where the weight of the twigs resulted to be 12% compared to the leaves. The whole material was completely soaked in 75 L of water, compressing the leaves with the non hermetic closure cover of the extraction tank. The temperature was raised under direct flame from GPL and controlled with a thermocouple immersed in the bath.

At the end of the bath heating, the stove flame was put off, it was left to slowly cool up to 60°C and then the collection of the extraction water was activated by opening the tap located at the bottom of the boiler. The organic mass of leaves and twigs was left to drain, then the tap at the bottom of the boiler was closed again, and new fresh demineralized water was added in order to proceed to the second aqueous hot extraction bath, carried out at the conditions described above.

For a quantitative extraction of the active principles at least two phases of hot extraction are necessary, wherein the second extracts more polyphenols compared to the first, which has the function of soaking the skin rich in waxes of the leaves.

After the drain phase, the leaves still contained water, evaluated to about 12% of the dry weight of the starting leaves. The extraction waters presented a yellow-green color in a rather cloudy and opalescent aqueous medium. They were pre-filtered with a 90 µm inox sieve, which allows to eliminate leaves fragments, deposits and soil from the filtrate, and the filtrate liquid was subjected to analysis.

The results of such analysis, in terms of chemical composition, were similar to those presented in the previous example, specifically at the columns ₁^{a} and 3^{a} of **Table 2.**

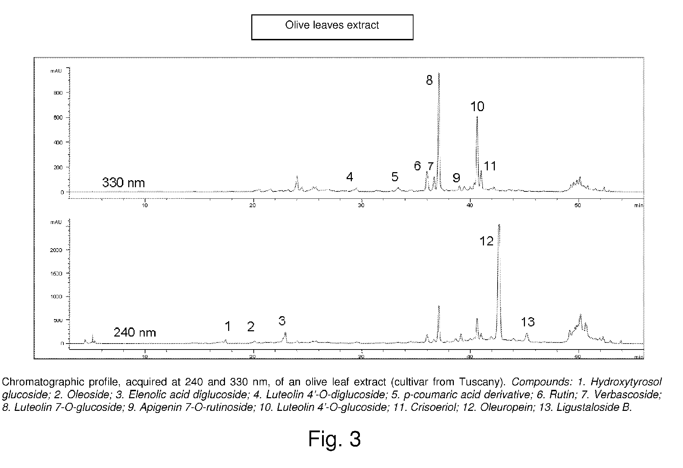

**Figure 3** of the attached drawings shows the HPLC chromatographic profile, taken at 240 and 330 nm, of an extract from fresh olive tree leaves and twigs obtained according to the present embodiment example. The identity of the compounds corresponding to the various peaks of the two chromatograms are shown in the legenda of the same figure.

### EXAMPLE 3

### Ultrafiltration and reverse osmosis of the pre-filtered extract from fresh leaves and twigs (UF + RO)

According to the procedure of the invention, the aqueous extract from olive tree pruning wastes, produced and filtered as in the previous example, is poured again in the feeding tank, respectively of the MF or UF apparatuses, which have a specific system for cooling, by a heat exchanger fed by a chiller. The latter allows to keep the tangential filtration temperature to the desired levels (20-30 °C).

The UF process has been realized using polymeric modules having spiral shaped geometry, in particular modules 4" x 10" in polyethersulphone from Osmonics (USA), with molecular cut-off of 6 kDa, spacer 28 mil and filtering surface of 8.36 m². Further, the permeate of UF has been concentrated in reverse osmosis (RO) into a pilot plant with polymeric spiral shaped module by DE SAL (USA) company in composite polyamide having filtering surface of 7.0 m².

In the present example, 23 kg of fresh leaves and twigs from pruning have been used, in two batches of about 75 L (total 150 L) of demineralized water (30% dry weight per batch with respect to water), from which an extract of the same type of the Example 1 has been achieved. The latter was in turn subjected to mechanical pre-filtration. The clarified liquid was fed to ultrafiltration, and the permeate from this phase was treated by reverse osmosis.

The water obtained as permeate from RO was used for a second hot extraction from olive tree leaves. The RO concentrate represents the final product of the process, as it is rich of polyphenols.

The distribution of the aqueous volumes in the total treatment process (UF + RO) is reported in the following table.

**Table 3**

| Volumetric distribution of the fractions treated with UF + RO techniques | |
|---|---|
| Liquid fraction | Volumes (L) |
| Infusion volume (two batches) | 240 (in) |
| UF permeate | 210+20 (out) |
| Diafiltration volume | 20 (in) |
| UF retentate | 30 (out) |
| VCR¹ | 10.43 |
| RO feed | 230 (in) |
| RO permeate | 200 (out) |
| RO concentrate | 28 (out) |
| VCR¹ | 8.21 |

| | |
|---|---|
| ¹: VCR: volumetric concentration ratio | |

The process parameters of the UF test are reported in **Table 4** below.

**Table 4**

| Process parameters of the 6 kDa UF test | |
|---|---|
| Process parameter | Value |
| Feed flow rate | 4.4 m³/h |
| Trans-membrane pressure | 4.7 bar |
| Temperature | 22-24 °C |
| Scroll speed rate | 0.5 m/s |
| Reynolds number | 1900 |
| VCR¹ | 10.43 |

| | |
|---|---|
| ¹: VCR: volumetric concentration ratio | |

Once achieved a VCR of about 4.1, 21 liters of osmotized water have been added to the 24 liters of concentrate (+hold up volume) respectively four times (total water added 84 liters), continuing the filtration (UF/DF) in order to increase the yield. 95 liters of permeate of 6 kDa UF/DF and 13 liters + holdup volume of concentrate of UF/DF 6 kDa have been achieved overall.

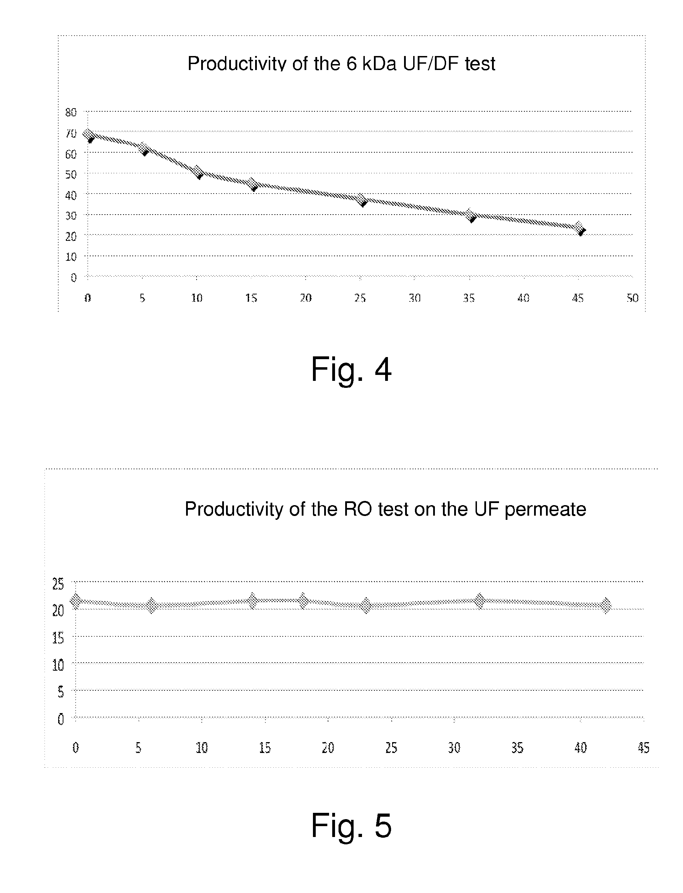

The diagram attached here as **Figure 4** shows the productivity curve, in terms of liters produced in time unit and for filtering surface unit, from the 6 kDa UF and UF/DF tests.

As already noted, the permeates of UF and those deriving from UF/DF have been treated in RO in order to concentrate the product rich in antioxidant substances and to eliminate the water as permeate. About 230 L of UF permeate have been fed to RO, operating under the conditions reported in the following table

**Table 5**

| Process parameters of the RO test | |
|---|---|
| Process parameter | Value |
| Feed flow rate | 650-600 m³/h |
| Working pressure | 20-26 bar |
| Temperature | 20-27°C |
| Permeate flow rate | 22 (L/m²h) |
| Salinity expressed in mg/L | 0-56 |
| VCR¹ | 18.8 |

| | |
|---|---|
| ¹: VCR volumetric concentration ratio | |

The pressure has been maintained at 20 bar, being able to raise up to 26, automatically in the plant. The RO test has had an overall duration of 43 minutes, wherein any loss of module permeability has been noted. This fact means that the membrane under examination may operate, without washing, for long periods (days). The throughput of the membrane remains constant around 22.0 L/m² hour, as the diagram of the attached Figure 5 shows.

The obtained concentrate of reverse osmosis has been further transformed in powder by spray drying process, using an atomizer for laboratory type ICF Lab 25, which has an evaporative capacity of 500 ml/h. During the experimentation the possibility to achieve solid extracts at different titers, by adding maltodextrins, dextrans or inert powders such as silica, compatibly with the proposed uses, has been studied.

### EXAMPLE 4

### Microfiltration and reverse osmosis of the pre-filtered extract from fresh leaves and twigs (MF + RO)

The aqueous extract from olive tree pruning wastes, again produced and filtered as in the Example 2, was subjected to microfiltration (MF) in place of the ultrafiltration. The MF gives the opportunity to operate with hot liquid at 80 °C, therefore without cooling the raw aqueous extract.

Tubular alumina ceramic membranes has been used, having an internal coating by zirconia, isoflux type by Tami (France), in a "sunflower" conformation (23 channels having diameter of 3.6 mm) and porosity of 0.14 µm and filtering surface of 0.35 m². The process has been carried out into a pilot plant of ENEA, which employs two ceramic membranes in parallel of the type of those above indicated.

The process parameters of the MF test are reported in the following table.

**Table 6**

| Process parameters of 0.14 µm MF test | |
|---|---|
| Process parameter | Valour |
| Feed flow rate | 9.5 m³/h |
| Transmembrane pressure | 1.35 bar |
| Temperature | 20-29 °C |
| Scroll speed rate | 5.6 m/s |
| VCR¹ | ca 3.5 |

| | |
|---|---|
| ¹: VCR: volumetric concentration ratio | |

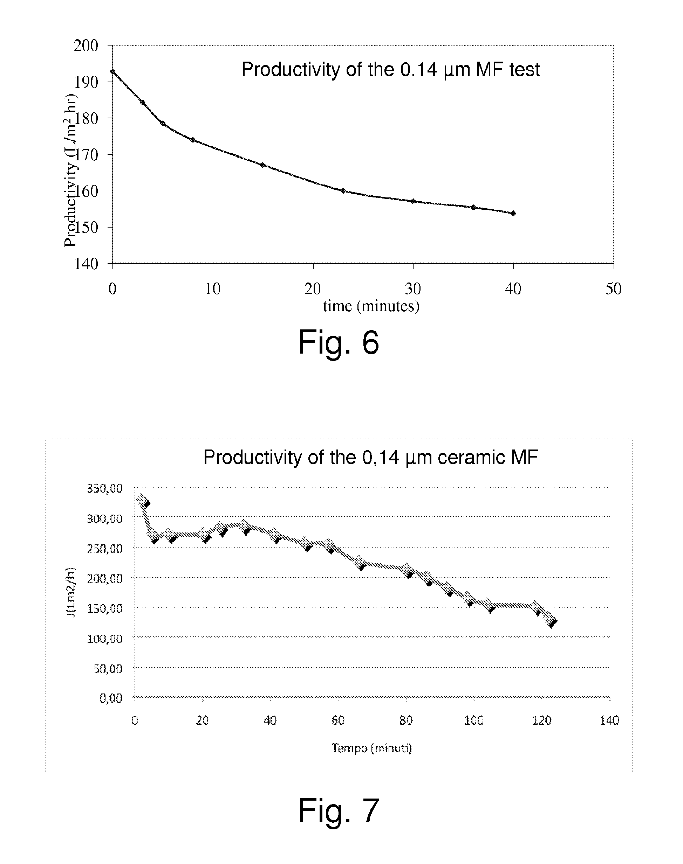

In the attached diagram of **Figure 6** the productivity curve is reported, in terms of liters produced per time unit and filtering surface, from the 0.14 µm MF test. The curve trend demonstrates that the ceramic MF technique guarantees a permeate flow rate considerably higher than UF (Figure 4). For instance at mid test, after 20 minutes, the MF productivity is of 165 L/m² h compared to 40 L/m² h by U F.

Once reached a VCR of about 3.5, respectively 23 liters of osmotized water (MF/DF) have been added to the 28 liters of concentrate of MF 0.14 µm, and continuing the filtration, in order to increase the yield of the molecule of interest in the permeate. In 0.14 µm MF/DF 26.5 liters of permeate were obtained. The final volume of the concentrate of 0.14 µm MF/DF is about 25 liters. The average flow rate of permeate in DF rises to values of about 170 L/m²h_{.}

26 liters of permeate of DF were added to the permeate of MF, and this solution of 97 L, having an intense green color, was treated by RO. The flow rate of permeate of this test is identical to the previous one, having an average productivity of about 20 L/m²h at 22 bar of transmembrane pressure.

### EXAMPLE 5

### Microfiltration and reverse osmosis of the pre-filtered extract of fresh leaves and twigs (MF + RO)

The same hot extraction protocol of polyphenols from fresh leaves and twigs of Example 1 has been repeated After the pre-filtration with a 90 µm woven metal wire cloth sieve the aqueous extract was treated by ceramic 0.14 µm MF, while the permeate was treated by RO, according to the same procedure of Example 4.

In the following **Table 7** the volumes distribution in the MF + RO test is reported, including the diafiltration volume of 48 L used to increase the extraction of polyphenols in the MF phase.

**Table 7**

| Volumetric distribution of fractions treated with MF+RO technique | |
|---|---|
| Liquid fraction | Volumes (L) |
| Infusion volume (two batches) | 357.5 (in) |
| MF permeate | 325 (out) |
| Diafiltration volume | 48 (in) |
| MF retentate | 32 (out) |
| VCR¹ | 8.94 |
| RO feed | 373 (in) |
| RO permeate | 345 (out) |
| RO concentrate | 35 (out) |
| VCR¹ | 9.85 |

| | |
|---|---|
| ¹: VCR: volumetric concentration ratio | |

In the diagram of the attached **Figure 7** the trend of the productivity of the MF test of the present example is reported, in terms of liters produced over time unit and per filtrating surface unit.

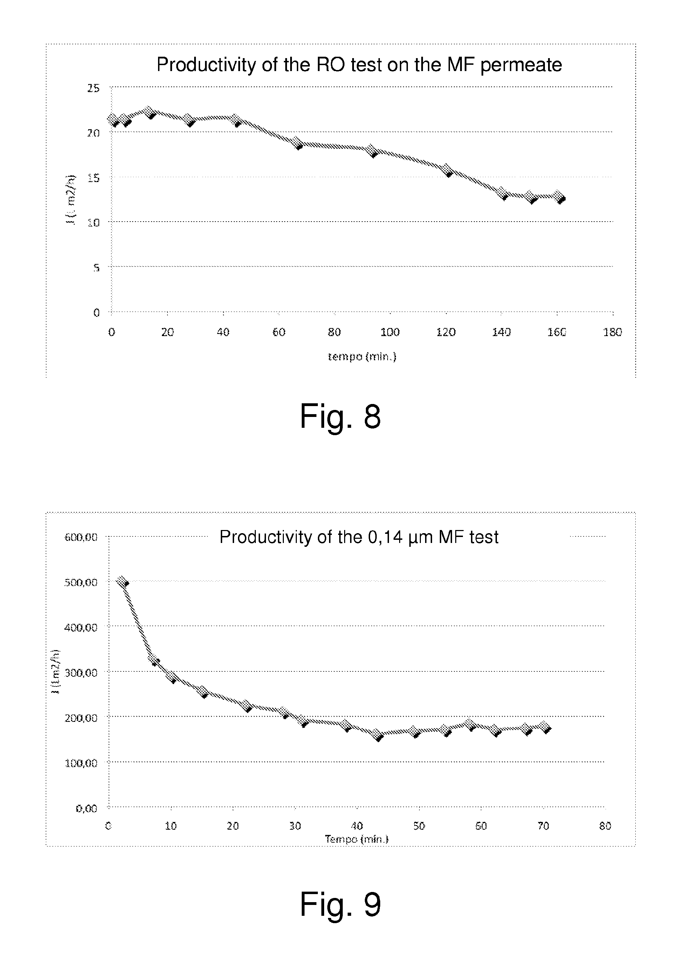

Further, the diagram of **Figure 8** shows the trend over time of the RO permeate flow rate, operating at an average pressure of 22 bar, and between the start and the end of the test itself. As it may be noticed from the figure, the productivity of RO does not change significantly in the example reported below.

The reverse osmosis concentrate obtained was then analyzed in order to determine the overall contents of bio-phenols and the concentration of any single active principle. Each molecule has been singularly quantified being possible to define the extract titer as total secoiridoidic derivatives, as total flavonoids, as verbascoside and total cinnamic acids, apart from the total of the present polyphenols. The analytical results obtained by HPLC/DAD, expressed either in g/L of the single present compounds in the RO concentrate either in mg/g of powder on the spry drying product, are reported in the following table.

**TABLE 8**

| Compound analysis present in the RO concentrate and in the corresponding product in powder after spray-drying (HPLC/DAD) | | |
|---|---|---|
| | **RO concentrate** | **RO concentrate spray dried** |
| | g/L | mg/g |
| | | |
| Hydroxytyrosol glucoside | 0.01 | 19.14 |
| Hydroxytyrosol | 0.94 | 4.30 |
| Oleoside (demethyl AE gluc.) | 0.52 | 9.84 |
| Elenolic acid glucoside | 0.54 | 10.55 |
| | | |
| Caffeic acid | 0.01 | 0.15 |
| Caffeic acid derivatives | 0.01 | 0.32 |
| P-cumaric acid | 0.02 | 0.42 |
| P-cum. acid (or caff.) derivatives | 0.03 | 0.86 |
| P-cum. acid derivatives | | 0.08 |
| Verbascoside | 0.22 | 8.01 |
| | | |
| Luteolin diglucoside | | 0.55 |
| Taxifolin glucoside | 0.03 | 0.54 |
| Rutin | 0.21 | 4.57 |
| Luteolin 7-O-glucoside | 0.41 | 9.38 |
| Luteolin 4'-O-glucoside | 0.11 | 2.49 |
| Chrysoeriol 7-O-glucoside | | 1.05 |
| | | |
| Demethiloleuropein | 0.71 | 14.43 |
| Oleuropein | 313.98 | 168.62 |
| Ligustaloside B | 0.62 | 16.92 |
| | | |
| **TOTAL POLYPHENOLS** | **318.38** | **272.22** |

The preceding results show how it is possible, by the proposed procedure according to the invention, to achieve extracts with different contents in polyphenols compounds, both liquid or in powder.

The antiradical activity of the obtained product, that is correlatable to the antioxidant properties of the same, has been evaluated by spectrophotometry by the use of the stable radical DPPH and the application of spectrophotometric techniques. The tested extracts gave evidence to a percent average antiradical activity not lower than 70%. Furthermore, the results related to the RO concentrate dried by spray drying, the composition of which is reported in Table 8, show an EC₅₀ of 17.76 mM.

### EXAMPLE 6

### Treatment of fresh leaves extract by microfiltration, nanofiltration and reverse osmosis (MF + NF + RO)

According to the procedure proposed by the present invention, the MF or UF permeate may be further purified by nanofiltration techniques (NF) before to be sent to the Reverse osmosis (RO). Starting from an initial matrix consisting of fresh leaves and twigs, NF was employed on the MF permeate with the aim to concentrate oleuropein and also the molecules having molecular weight lower than 200 Da, yet using the RO technique.

Hot extraction tests were carried out from fresh leaves of carboncella (an olive tree cultivar originating in the central Italy) with the same extractive procedure of the preceding examples.

The volumes of the different fractions at play are reported in the following table.

**Table 9**

| Volumetric distribution of the fractions treated with MF+NF+RO techniques | |
|---|---|
| Liquid fraction | Volumes (L) |
| Infusion volume (two batches) | 160 (in) |
| MF ceramic 0.14 µm permeate | 224.3 (out) |
| Diafiltration volume | 64 (in) |
| MF retentate | 45 (out) |
| VCR¹ | 5.00 |
| NF feed+ 62 dia | 241 (in) |
| NF permeate | 209 (out) |
| NF concentrate | 35 (out) |
| VCR¹ NF | 7.53 |
| RO feed | 209 |
| RO permeate | 25 |
| RO concentrate | 184 |

| | |
|---|---|
| ¹: VCR: volumetric concentration ratio | |

In the diagram of **Figure 9** the trend over time of the flow rate of permeate in the 0.14 µm MF ceramic test is reported.

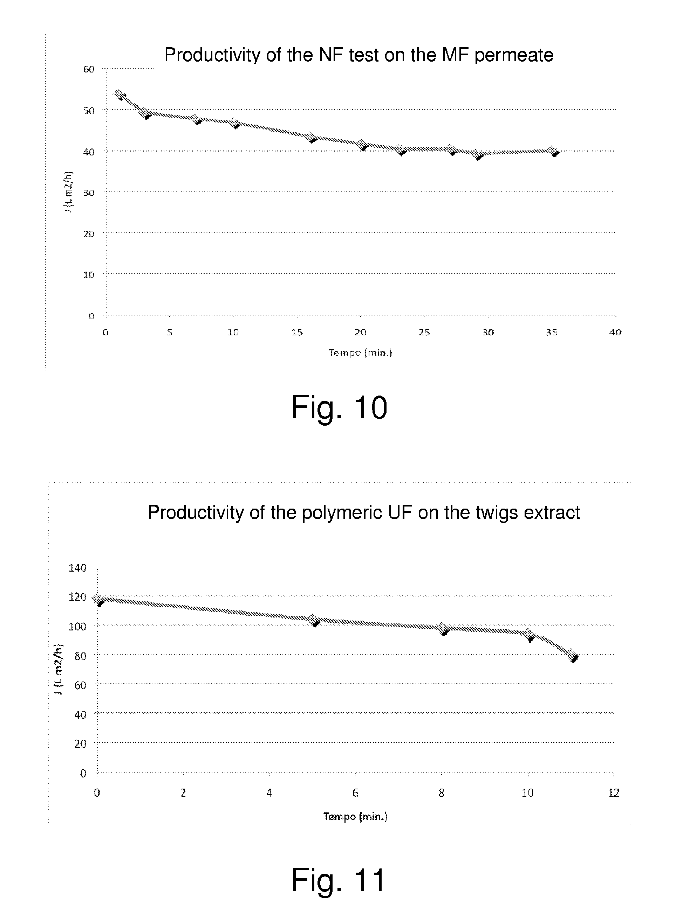

The MF permeate has been treated then by NF, using a spiral wound module for nanofiltration having molecular cut-off of about 200 Dalton in order to recover the oleuropein from retentate and to produce a permeate having bioactive molecules (polyphenols, anthocyans, flavans) having lower molecular weight. The productivity of the NF process, obtained at 11 bar, is reported in Figure 10 herein attached.

The NF permeate was treated by RO in order to concentrate the molecules of bioactive interest having low molecular weight. Flow rate data of the RO test over time arenot reported because they are identical those obtained in all the RO tests presented above.

### EXAMPLE 7

### Treatment of hot extract from pruning twigs by microfiltration (or ultrafiltration) and reverse osmosis (MF/UF + RO)

The same protocol for hot extraction of polyphenols from fresh leaves and twigs of Example 1 was repeated on a fed of only olive tree twigs. In this case the weight of the biomass was 12% compared to water. After the pre-filtration the fed was sent to a polymeric membrane having a spiral wound conformation wrapped in polysulphone, having a molecular cut-off of 100 kDa and having a filtering surface of 5 m². It is a borderline membrane between UF and MF, that will be defined as polymeric UF.

The volume of the different fractions at play are reported in the following table.

**Table 10**

| Volumetric distribution of the fractions treated with UF polymeric + RO techniques | |
|---|---|
| Liquid fraction | Volumes (L) |
| Infusion volume (two batches) | 130 (in) |
| UF permeateF | 24 (out) |
| Diafiltration volume | 20 (in) |
| UF retentate | 32 (out) |
| VCR¹ | 7.00 |
| RO feed | 150 (in) |
| RO permeate | (out) |
| RO concentrate | 35 (out) |
| VCR¹ 1 | 9.85 |

| | |
|---|---|
| ¹: VCR: volumetric concentration ratio | |

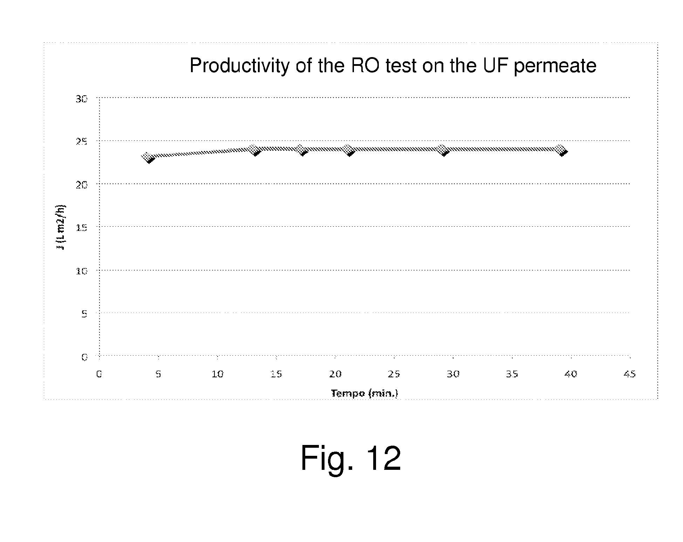

The time trend of the permeate flow rate of the polymeric UF membrane is reported in the diagram of Figure 11 herein attached, meanwhile the time trend of the permeate flow rate through the RO membrane, related to the reverse osmosis test carried out on the UF permeate, is shown in **Figure 12****.**

It is to be noted that in the reverse osmosis test carried out on a material exclusively consisting of twigs, working in the same operation conditions as all the RO operations carried out previously, a higher flow rate of permeate is achieved. This fact is to be ascribed to the dilution of the treated matrix, deriving from a weight reduction of the biomass compared to the distilled water (12% compared to 20%-30%).

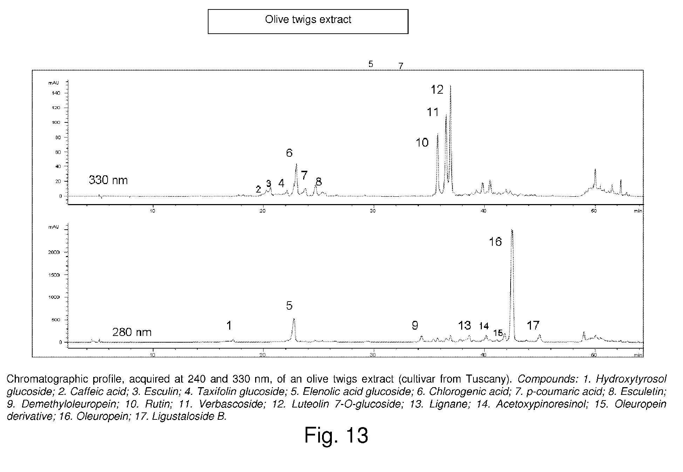

The aqueous extracts obtained by infusion from olive tree twigs according to the first part of the present embodiment example were analyzed by HPLC. Figure 13 of the attached drawings show the relative chromatographic profile, acquired at 240 and 330 nm. The identities of the corresponding compounds at the various peaks of the two chromatograms are indicated in the explanation of the same figure. As it is possible to note yet from Figure 13, the chromatographic analysis evidenced the oleuropein is the main compound contained in the twigs extract. Such result is confirmed by the following Table 11, which reports the quantitative data of the different polyphenols classes present in the olive tree twigs of two different Italian varieties.

**Table 11**

| Analysis of the compounds present in olive twigs aqueous extract (HPLC/DAD) | | | | |
|---|---|---|---|---|
| | Variety A Twigs | | Variety B Twigs | |
| | (mg/g) | % | (mg/g) | % |
| OH-tyrosol and derivatives | 0.5 | 1.4 | 1.3 | 11.3 |
| Elenolico acid derivatives | 5.4 | 15.4 | 1.6 | 14.4 |
| Oleuropeina and derivatives | 22.3 | 63.3 | 5.0 | 44.4 |
| Lignans | 4.7 | 13.4 | 1.3 | 12.0 |
| Coumarins | 0.2 | 0.5 | 0.4 | 3.5 |
| Flavonoides | 1.8 | 5.1 1 | 1.4 | 12.2 |
| Verbascoside and cinnamic acid | 0.3 | 0.9 | 0.2 | 2.1 |
| **Total Polyphenols** | **35.2** | | **11.2** | |

As it is possible to notice from the preceding data, in one of the cultivar, even if the oleuropein and derivatives represent always the more representative class in the twigs extract, it is possible to notice the presence of higher percentage of other classes, differing from what observed in the other variety. In particular the flavonoids, the lignans and the coumarins are present at percentage of 12%, 12% and 3.5% respectively.

After the polymeric UF operation, the UF permeate was fed to the reverse osmosis according to the procedures as expected from the invention, and the concentrate of reverse osmosis obtained was been further analyzed. The analytical results achieved by HPLC/DAD, expressed both in g/L of the singular compound present in the RO concentrate or in mg/g of powder on the spray drying product, are reported in the following table.

**TABLE 12**

| Composition of RO concentrate from pruning twigs and of the corresponding powder product after spray-drying (HPLC/DAD) | | |
|---|---|---|
| | **RO Concentrate spray dried** | **RO Concentrate** |
| | mg/g dust | mg/L |
| Hydroxytyrosol glucoside | 8.98 | 279.84 |
| Oleoside (Demethyl AE gluc.) | 5.73 | 180.36 |
| Elenolic acid glucoside | 1.55 | 52.65 |
| Lignan (Pinoresinol ) | 6.19 | 176.90 |
| Acetoxypinoresinol | 7.96 | 274.51 |
| Taxifolin glucoside | 0.57 | 18.36 |
| Taxifolin derivative | 2.45 | 78.00 |
| Taxifolia aglicone | 2.49 | 73.00 |
| 10 OH oleur gluc | 2.88 | 94.45 |
| Demethyl oleuropein | 1.71 | 54.03 |
| Oleuropein diglucoside | 1.34 | 39.13 |
| Oleuropein | 35.46 | 1187.70 |
| Ligustaloside B | 2.82 | 91.20 |
| chlorogenic acid | 0.00 | 0.00 |
| Verbascoside | 7.66 | 239.54 |
| Esculin | 0.72 | 22.40 |
| Esculetin | 1.65 | 55.12 |
| Quercetin ramnoside | 0.20 | 6.79 |
| Quercetin aglicone | 0.20 | 7.20 |
| **TOTAL POLYPHENOLS** | **90.55** | **2931.18** |

It is to be noted that the titer in bioactive components of the preceding extracts may be expressed either as total content achieved by spectrophotometry, either as content in the different sub-classes evaluable by HPLC/DAD. As previously described, each fraction may be further evaluated by the specific antiradical activity by the use of DPPH, correlatable with the antioxidant properties of the same extract.

### EXAMPLE 8

### MEMBRANES WASHING CYCLE

At the end of a working cycle the membrane modules are re-conditioned in order to carrying the following working cycle. This fact allows to remove the eventual sediment occurring on the membranes, so as to reset the better permeate flows, i.e. the productivity of these filters. For the membranes of MF, UF, NF and of RO used in the Examples 3-7 above the following procedure have been adopted.

In order to wash the MF ceramic membrane a rinsing of the module is carried out using tap water, for 5 minutes, followed by a chemical washing with a basic solution (soda 0.5 M) at 35°C for 25 minutes continuously. A second washing is carried out using water for eliminating the chemical reactive, up to the neutralization of the washing solution.

For the UF membrane, a rinsing is carried out with tap water for 5-10 minutes, without recirculation, followed by a treatment with soda 0.25 M for 15 minutes; at the end it is washed with tap water and controlled of the permeability of the module are carried out.

For the RO membrane and for that of NF the washing protocol simply includes a rinsing with tap water, followed by a washing with distilled water.

### EXAMPLE 9

### Treatment of wet pomace residues from two-phase processes (second matrix)

A further extraction and fractioning process analogous to the one described for the pruning is applied to the wet pomace from two-phase processes.

A pre-dilution with acid solution at pH 3 is necessary in order to stimulate the extraction of the biocomponents and to stabilize the chemical environment conditions. The cold solution obtained with such a dilution is subjected to a pre-treatment and then to the MF, NF or RO process as described for the treatment of fresh olive leaves.

The following **Table 13** reports the average compositions of the starting matrices after pre-treatment with cold extraction acid.

**TABLE 13**

| Composition of the cold extract, after acid treatment, of wet pomace from two-phase process | | |
|---|---|---|
| | **Extract in** | |
| | **H₂O** | **EtOH/H₂O** |
| | g/L | g/L |
| Oleoside gluc deriv | 0.564 | 0.255 |
| Oleoside gluc | 1.999 | 1.15 |
| Elenolico acid (EA) | 0.363 | 0.205 |
| OH-Tyr glic | 0.300 | 0 |
| OH-Tyr derivatives | 0.133 | 0.195 |
| OH-Tyr (hydroxytyrosol) | 0.157 | 0.24 |
| OH-Tyr gluc | 0.134 | 0 |
| Tyr (tyrosol) | 0.100 | 0.105 |
| Tyr gluc | 0.000 | 0 |
| Tyr derivatives | 0.024 | 0 |
| Vanillic acid | 0.000 | 0 |
| Demethyl oleurop | 0.000 | 0 |
| Secoiridoidic deriv | 0.493 | 0 |
| DACOLAG | 0.384 | 1.925 |
| Oleocanthal | 0.228 | 0 |
| Caffeic acid derivatives | 0.027 | 0 |
| Caffeic acid | 0.000 | 0 |
| P-coumaric acid | 0.000 | 0 |
| bOH verbascoside (isomers) | 1.782 | 3.5 |
| Verbascoside | 0.034 | 4.405 |
| lsoverbascoside | 0.045 | 0.215 |
| Caffeoyl ester secologanoside | 0.051 | 0.065 |
| p-coumaroil ester secologanoside | 0.109 | 0.2 |
| **Total** | **6.928** | **8.63** |
| OHTyr and derivatives | 1.601 | 1.81 |
| caffeic/verbascoside derivatives | 1.920 | 3.6 |
| AE derivatives | 2.927 | 2.46 |
| OHTyr and derivatives % | 23.81 | 20.97 |
| caffeic/verbascoside derivatives% | 23.15 | 41.71 |
| AE derivatives% | 46.72 | 28.51 |

The qualitative and quantitative composition of the permeate of ultrafiltration (PUF) obtained by the wet pomace extract described above is reported in the following table. The ultrafiltration was carried out using operational conditions analogous to those of Example 3.

**TABLE 14**

| Composition of the ultrafiltration permeate of wet pomace extract from two-phase process | |
|---|---|
| | **PUF** |
| | g/L |
| Oleoside gluc deriv | 0.451 |
| Oleoside gluc | 1.600 |
| Elenolico acid (EA) | 0.291 |
| OH-Tyr glic | 0.240 |
| OH-Tyr derivatives | 0.107 |
| OH-Tyr (hydroxytyrosol) | 0.125 |
| OH-Tyr gluc | 0.107 |
| Tyr (tyrosol) | 0.080 |
| Tyr gluc | 0.000 |
| Tyr derivatives | 0.019 |
| Vanillic acid | 0.000 |
| Demethyl oleurop | 0.000 |
| Secoiridoidic deriv | 0.394 |
| DACOLAG | 0.307 |
| Oleocanthal | 0.182 |
| Caffeic acid derivatives | 0.022 |
| Caffeic acid | 0.000 |
| P-coumaric acid | 0.000 |
| bOH verbascoside (isomers) | 1.425 |
| Verbascoside | 0.027 |
| lsoverbascoside | 0.036 |
| Caffeoyl ester secologanoside | 0.041 |
| p-coumaroil ester secologanoside | 0.087 |
| **Total** | **5.543** |
| OHTyr and derivatives | 1.28 |
| caffeic/verbascoside derivatives | 1.54 |
| AE derivatives | 2.34 |
| OHTyr and derivatives % | 19.046 |
| caffeic/verbascoside derivatives% | 18.520 |
| AE derivatives% | 37.373 |

### EXAMPLE 10

### Treatment of destoned pigmented olive pulps (third matrix)

Starting from the pigmented olives (for ex., from plants of cv. Carboncella or of cv. Leccino) as starting material, the optimized process to obtain the corresponding fraction includes, subsequently to the destoning of the same olives, an aqueous extraction at acid pH (pH 3 for hydrochloric acid) in order to stabilize the anthocyanosidic components derived from cyanidine.

The following **Tables 15** and **16** show the details of an aqueous extract of destoned pigmented olive pulps of a local variety of the Sabina, Lazio, Italy, acidified at pH 2.5 by hydrochloric acid.

**TABLE 15**

| Composition of an aqueous extract acidified to pH 2.5 of pigmented olive pulps | |
|---|---|
| | **mg/g olive** |
| Hydroxytyrosol glycol | 0.00 |
| Hydroxytyrosol glucoside | 0.00 |
| Hydroxytyrosol | 0.01 |
| Hydroxytyrosol derivatives | 0.00 |
| Oleoside (demethyl EA gluc.) | 0.20 |
| Oleoside (demethyl EA gluc.) derivatives | 0.38 |
| Demethyl AE glucoside | 0.00 |
| Elenolic acid glucoside | 0.35 |
| Elenolic gluc der | 0.22 |
| Caffeic acid derivatives | 0.00 |
| P-coumaric acid derivatives | 0.00 |
| Verbascoside | 0.17 |
| Luteolin diglucoside (30 min) | 0.00 |
| Rutin | 0.41 |
| Luteolin ramn gluc | 0.11 |
| Luteolin 7-O-glucoside | 0.09 |
| Quercetin 3-O-glucoside | 0.00 |
| Apigenin 7-O-glucoside | 0.00 |
| Luteolin 4'-O-glucoside | 0.00 |
| Crhysoeriol 7-O-glucoside | 0.00 |
| Luteolin der | 0.03 |
| Luteolin aglicone | 0.02 |
| Demethyloleuropein | 0.00 |
| 10 OH oleur gluc | 0.00 |
| Oleuropein | 0.36 |
| Oleuropein der | 0.74 |
| Ligustaloside B | 0.00 |
| Oleuropein aglicone der | 2.87 |
| Anthocianosides (cyanidin glucosides) | 13.71 |
| **TOTAL POLYPHENOLS** | **19.66** |

**TABLE16**

| Aqueous extract of pigmented olive pulps acidified to ph 2.5 Data expressed for polyphenol classes | |
|---|---|
| | **average values mg/g olives** |
| Hydroxytyrosol derivatives | 0.01 |
| Elenolic acid derivatives | 0.01 |
| Hydroxycinnamic derivatives | 0.00 |
| Verbascoside | 0.17 |
| Flavonoides | 0.17 |
| Oleuropein derivatives | 3.96 |
| Ligustaloside B | 0.00 |
| Anthocianosides (cyanidin glucosides) | 13.71 |
| **TOTAL POLYPHENOLS** | **18.04** |

Subsequently the cold extraction treatment and to the mechanical filtration of the extract an UF is operated analogously to what described for the Examples 3 and 9. The qualitative and quantitative composition of the obtained extract is reported in the following table.

**TABLE 17**

| Composition of the extract from pigmented olive pulps after ultrafiltration | |
|---|---|
| | **mg/L extract** |
| Hydroxytyrosol glicol | 0.00 |
| Hydroxytyrosol glucoside | 0.00 |
| Hydroxytyrosol | 3.08 |
| Hydroxytyrosol der | 0.00 |
| Oleoside (Demethyl EA gluc.) | 49.14 |
| Oleoside (Demethyl EA gluc.) der | 95.08 |
| Demethyl EA diglucoside | 0.00 |
| Elenolic acid glucoside | 88.87 |
| Elenolic acid gluc der | 55.47 |
| Caffeic acid derivatievs | 0.00 |
| P-coum. acid derivatives | 0.00 |
| Verbascoside | 43.62 |
| Luteolin diglucoside (30 min) | 0.00 |
| Rutin | 101.61 |
| Luteolin ramn gluc | 26.16 |
| Luteolin 7-0-glucoside | 22.08 |
| quercetin 3-0-glucoside | 0.00 |
| apigenin 7-0-glucoside | 0.00 |
| Luteolin 4'-O-glucoside | 0.00 |
| Chrysoeriol 7-0-glucoside | 0.00 |
| Luteolina der | 7.85 |
| Luteolina aglicone | 4.52 |
| Demethyloleuropeina | 0.00 |
| 10 OH oleur gluc | 0.00 |
| Oleuropein | 89.35 |
| Oleuropein der | 185.32 |
| Ligustaloside B | 0.00 |
| Oleuropeina aglicone der | 716.00 |
| Anthocianosides (cyanidin glucosides) | 694.47 |
| **TOTAL POLYPHENOLS** | **2182.62** |

**TABLE 18**

| Composition of the extract from pigmented olive pulps after ultrafiltration. Data expressed for polyphenol classes | |
|---|---|
| | **Average values extract mg/L** |
| Hydroxytyrosol derivatives | 3.08 |
| Elenolic acid derivatives | 288.56 |

| | **mg/L extract** |
|---|---|
| Hydroxycinnamic derivatives | 0.00 |
| Verbascoside | 43.62 |
| Flavonoides | 162.24 |
| Oleuropein derivatives | 990.66 |
| Ligustaloside B | 0.00 |
| Anthocianosides (cyanidin glucosides) | 694.47 |
| **TOTAL POLYPHENOLS** | **2182.63** |

For the study of the produced fraction from pigmented olive pulps the HPLC/DADA/MS qualitative and quantitative analysis conditions of the anthocyanosidic, cyanidine glucosides compounds, present in the olive pulp, have been optimized . With this aim, reverse phase column C18 and acid solvents at pH 2 for HCOOH were used, both as acidified water and as mixture of acid water/methanol and as acid water/acetonitrile mixtures

### EXAMPLE 11

### Fractioning, enrichment and isolation of polyphenol compounds by use of resins

For the phase of refining the actives obtained from the fractioning by membrane filtration techniques, the following resins were experimentally used: XAD-4, XAD-7 HP and MN202 (Rohm and Haas Co.), with the aim to achieve fractions containing specific phenolic classes and isolated single active principles.

In the following **Table 19** the titers of the extracts obtained from fresh olive tree leaves and twigs after treatment with two different resins are reported (first matrix).

**TABLE 19**

| Composition of extracts from fresh olive tree leaves and twigs after treatment with two different resins | | |
|---|---|---|
| | **desorbed XAD4** | **desorbed XAD7HP** |
| | mg/g | mg/g |
| | | |
| Hydroxytyrosol glucoside | 84.55 | 12.56 |
| Hydroxytyrosol | 6.92 | 1.10 |
| Oleoside (Demethyl EA gluc.) | 5.98 | 0.90 |
| Elenolico acid glucoside | 4.93 | 0.94 |
| | | |
| Caffeic acid | 0.18 | 0.09 |
| Caffeic acid derivatives | 0.41 | 0.19 |
| P-coumaric acid | 0.37 | 0.29 |
| P-cum. acid (o caff.) derivatives | 0.53 | 0.54 |
| P-cum. acid derivative | 1.49 | 0.52 |
| Verbascoside | 5.08 | 7.26 |
| | | |
| Luteolin diglucoside | 0.30 | 1.05 |
| Taxifolin glucoside | 1.06 | 1.01 |
| Rutin | 4.50 | 7.07 |
| Luteolin 7-0-glucoside | 12.55 | 17.67 |
| Luteolin 4'-O-glucoside | 3.58 | 5.37 |
| Chrysoeriol 7-0-glucoside | 1.07 | 2.31 |
| | | |
| Demethyloleuropein | 4.25 | 13.28 |
| Oleuropein | 77.54 | 240.37 |
| Ligustaloside B | 14.88 | 20.87 |
| | | |
| **TOTAL POLYPHENOLS** | **230.17** | **333.40** |

The two resins perform in a different way; for instance two different extracts were obtained not only from a point o view of the total amount, but also with different percents of the single type of present polyphenols.

The powder from XAD7HP has a titer of 33% in bioactive molecules very superior to the titers of 6-12% available on the market, and of 24% in oleuropein.

The resin XAD4 seems to be more selective to the hydroxytyrosol and to its derivatives, with a titer of 9% in these molecules comparing to the 2% of the XAD-7HP.

In order to ascertain the specific selectivity of the used resins, over these a test was carried out with an aqueous solution of 3,4 dihydroxybenzoic acid standard (protocatechuic acid). Such standard has been selected on the basis of its chemical features (presence of a catecolic group), which represent the features of the compounds of major interest present in the subject matrix. The molecules which present a catecol, as hydroxytyrosol, are characterized by high antioxidant activity.

The results obtained with this standard are reported in the following **Table 20**. As far as it concerns this molecule big differences have not been noticed either in adsorbance or desorbance among the three studied resins, XAD4, XAD7 and MN202. The resin MN202 results to show the maximum affinity towards the 3,4 dihydroxybenzoic acid, for instance it absorbs about the 100%, then completely desorbed eluting the resin with ethanol.

**TABLE 20**

| Evaluation of the selectivity towards 3,4 dihydroxybenzoic acid of three different polymeric resins (values expressed in area subtended to the chromatographic peak | | | | |
|---|---|---|---|---|
| | 3,4OHbenzoic (area) | Starting diference-eluate (adsorbed) | %adsorb | %desorb |
| Starting standard value | 18191 | | | |
| eluate MN202 | 54 | 18137 | 99.7 | 100.0 |
| eluate XAD4 | 1485 | 16706 | 91.8 | 98.6 |
| eluate XAD7 | 1046 | 17145 | 94.2 | 99.1 |
| desorbed MN202 | 18146 | | | |
| desorbed XAD4 | 16480 | | | |
| desorbed XAD7 | 16992 | | | |

Also the other two resins show a high adsorbance, so that the experiment using the matrix of interest results to be fundamental.

A test was performed also with a an aqueous solution of standard oleuropein (17,3 mg/ml, 32 mM), wherein 10 ml of solution have been adsorbed per gram of resin (0.32 mm of oleuropein/g resin). The resin XAD7 adsorbs the 99%, then desorbed at 96% with ethanol.

What previously described has been applied according to the integrated process proposed by the invention, in order to either increase the titer in polyphenols of at least 50%, either to increase the titer in oleuropein, when starting extracts from fresh leaves are used.

All the molecules identified in the experiment above reported are studied fir their activity of protection on proteins, lipids and DNA from oxidations due to free radicals, and for the effect anti-inflammatory and of inhibition of the platelet aggregation, the present demand for olive leaves and of herbal preparation obtained from *Olea europaea* is sustained overall by the antihypertensive action, hypocholesterol and hypoglycemic action linked to the present molecules.

A further interesting aspect is consisting by the obtaining of standardized extracts from olive twigs, which are product characterized, differently from the leaves extracts, by the presence of molecules of the class of lignans. As already noted, such compounds, differently from those of polyphenol nature, are reported in literature as natural phytoestrogens.

The liquid extracts according to the invention obtained from reverse osmosis concentration may have a direct utilization in the agro-alimentary sector as stabilizers, both for fresh products and for transformed products. As concerns the powder extracts, the latter may be employed as semi-finished products in the cosmetic sector (for products such as soaps, creams, gels etc.) and the dermal cosmetic sector (pharmaceutical cosmetics). The phytotherapeutic sector represents a further field where the standardized extracts in powder can be employed as semi-finished products for obtaining OTC products (over the counter pharmaceutical products).

Synthesizing, the productive systems according to the invention is characterized by the following advantageous aspects:
it employs an innovative process allowing the recovery and re-use of all the chemical components obtainable both from olive pruning waste and from the solid oil mill residues, if desired by integrating them with matrices added on purpose, also originated from the *Olea europaea* plant;
◆ it respects the environment, by means of the integrated reuse of the secondary effluents, including osmotized water;
◆ it avoids the use in the extraction of organic solvents, which would penalize the use of the recovered actives in the pharmaceutical and food industries;
◆ it allows to develop an industrial process on a productive scale coherent with the amounts of starting materials available on the national territory;
◆ it allows to obtain a pool of active principles purified, concentrated and formulated in a stabilized form.

The present invention has been disclosed with particular reference to some specific embodiments thereof, but it should be understood that modifications and changes may be made by the persons skilled in the art without departing from the scope of the invention as defined in the appended claims.

## Claims

1. A process for producing concentrated actives and/or refined extracts from tissues and byproducts of *Olea europaea* comprising a first section operating starting from leaves and twigs, a second section operating starting from wet pomace produced by two-phase oil extraction processes and a third section operating starting from destoned pigmented olive pulps, wherein the said first section comprises, in sequence, the following operations:
a) providing a first starting material consisting of olive leaves and twigs with a predetermined moisture content;
b) extracting active components from the solid matrix of the said first starting material with an aqueous or hydroalcoholic solvent;
c) treating the liquid phase resulting from the previous operation by tangential microfiltration (MF) or tangential ultrafiltration (UF), to give a retentate phase and a permeate phase;
d) treating the permeate resulting from the previous operation by reverse osmosis (RO), to give a retentate phase enriched in polyphenol active principles and a permeate phase consisting of demineralized water;
the wet solid or semisolid vegetal residue obtained from said extraction b) and from the possible phase of MF retentate from the operation c) being reusable as animal feedstock or for the production of fertilizers or energy, and said possible phase of UF retentate coming from the operation c) being enriched as well in polyphenol active principles,
and wherein said second and third sections each comprise at least a cold extraction treatment from the corresponding starting material, carried out in an acid aqueous solvent at a pH comprised between 1 and 6.

2. A process according to claim 1, wherein said first starting material provided in step a) consists of pruning waste and/or solid olive mill residues with fresh leaves, said RO retentate phase and said possible UF retentate phase being enriched in oleuropein.

3. A process according to claim 1, wherein said first starting material provided in step a) consists of pruning waste and/or solid olive mill residues previously treated in forced heated air for period of time sufficient to obtain drying of the leaves, said RO retentate phase and said possible UF retentate phase being enriched in flavonoids and hydroxycinnamic derivatives.

4. A process according to any one of claims 1-3, wherein said second section comprises, in sequence, the following operations:
A) providing a starting material consisting of wet pomace produced by two-phase oil extraction processes, diluted with water in an amount sufficient to obtain a semisolid consistence, and acidifying to a pH comprised between 2.5 and 4;
B) cold extraction, with an aqueous solvent, of active components from the solid matrix of said second starting material;
C) treating the liquid phase resulting from the previous operation by tangential microfiltration (MF) or tangential ultrafiltration (UF), to give a retentate phase and a permeate phase;
D) treating the permeate resulting from the previous operation by reverse osmosis (RO), to give a retentate phase enriched in polyphenol active principles and a permeate phase consisting of demineralized water;
the wet solid or semisolid vegetal residue obtained from said extraction B) and from the possible phase of MF retentate from the operation C) being reusable as animal feedstock or for the production of fertilizers or energy, and said possible phase of UF retentate coming from the operation C) being enriched as well in polyphenol active principles.

5. A process according to any one of claims 1-4, wherein said third section comprises, in sequence, the following operations:
i) providing a starting material consisting of destoned pigmented olive pulps, and acidifying to a pH comprised between 2.5 and 4;
ii) cold extraction, with an aqueous solvent, of active components from the solid matrix of said third starting material;
iii) treating the liquid phase resulting from the previous operation by tangential microfiltration (MF) or tangential ultrafiltration (UF), to give a retentate phase and a permeate phase;
iv) treating the permeate resulting from the previous operation by reverse osmosis (RO), to give a retentate phase enriched in anthocyanoside components, oleuropein and other polyphenol active principles and a permeate phase consisting of demineralized water the wet solid or semisolid vegetal residue obtained from said extraction ii) and from the possible phase of MF retentate from the operation iii) being reusable as animal feedstock or for the production of fertilizers or energy, and said possible phase of UF retentate coming from the operation iii) being enriched as well in anthocyanoside components, oleuropein and other polyphenol active principles.

6. A process according to claim 5, wherein downstream of said operations c), C) and/or iii) of tangential microfiltration (MF) or tangential ultrafiltration (UF), the following operation is carried out:
c1) treatment of the permeate resulting from the previous operation by tangential nanofiltration (NF), to give a retentate phase and a permeate phase, the permeate being fed to said phase d), D) or iv).

7. A process according to any one of claims 1-6, wherein said operation b) of extraction with an aqueous or hydroalcoholic solvent is an operation of hot infusion, carried out in an extraction bath at temperatures comprised between 60 °C and 95 °C for periods of time comprised from 15 to 60 min., and is followed by an operation of:
b1) mechanical separation of the solid material obtained from the previous operation, by filtration,
said operations b) and b1) being repeated at least a second time in the event that a more thorough extraction of said active principles is desired, the filtrate obtained being fed to the following operation c).

8. A process according to any one of claims 1-6, wherein said operation b) of extraction with an aqueous or hydroalcoholic solvent is an operation of pneumatic extraction under pressure, which alternates a dynamic step, obtained under a programmed pressure, with a static step, wherein the extractable substance is transferred into the aqueous phase, said operation thus affording a clear liquid extract, which is fed to the following operation c).

9. A process according to claims 7 or 8, wherein the weight/volume ratio of leaves and twigs fed to said operation b) and water or hydroalcoholic solution is from 10% to 35%.

10. A process according to claims 7 or 8, wherein each of said operations B) or ii) of cold extraction is followed by an operation of:
b1) mechanical separation of the solid material obtained from the previous operation, by filtration,
the filtrate obtained being fed to the following operation C) o iii).

11. A process according to any one of claims 1-10, wherein said operations c), C) and iii) of tangential microfiltration are carried out by means of spiral-shaped polymeric membranes or by means of tubular ceramic membranes having a molecular cut-off in the range of 0,10-3,0 µm.

12. A process according to any one of claims 1-10 wherein said operations c), C) and iii) of tangential ultrafiltration are carried out by means of spiral-shaped polymeric membranes having a molecular cut-off in the range of from 500 Da to 50 kDa.

13. A process according to claims 11or 12, wherein downstream of said operations c), C) and iii) of tangential microfiltration or ultrafiltration a diafiltration operation is carried out, by feeding the membrane with demineralized water obtained from said operation d), D) or iv) of reverse osmosis, which is added to the MF retentate or to the UF retentate.

14. A process according to any one of claims 1-13, wherein said operations d), D) or iv) of reverse osmosis are carried out by means of spiral-shaped polymeric membranes with high or low saline rejection, at a working pressure in the range of from 7 to 35 bar, in the temperature range of 5° to 60 °C.

15. A process according to any one of claims 1-14, wherein said operation c1) of tangential nanofiltration is carried out by means of spiral-shaped polymeric membranes having a molecular cut-off in the range of from 150 Da to 250 Da.

16. A process according to any one of claims 1-4, wherein said phases obtained as the retentate from said treatments d), D) and iv) of reverse osmosis, said phases obtained as the retentate from said treatments c), C) and iii) of ultrafiltration, when present, and said phases obtained as the retentate from said treatments c1) of nanofiltration, when present, are subjected to treatments of adsorption on chromatographic resins to give corresponding enriched and refined products.

17. A process according to claim 16, wherein said enriched and refined polyphenol products obtained by desorption from said resins and/or the aqueous fractions containing specific polyphenol classes not adsorbed on said resins are subjected to drying by spray-drying or freeze-drying.

18. A process according to claim 17, wherein said drying operation is carried out by spray-drying, performed with the addition of maltodextrins or dextrans.
